(19) Europäisches Patentamt / European Patent Office / Office européen des brevets

(11) **EP 3 950 711 A1**

(12) **EUROPEAN PATENT APPLICATION**
published in accordance with Art. 153(4) EPC

(43) Date of publication:
**09.02.2022 Bulletin 2022/06**

(21) Application number: **20784990.2**

(22) Date of filing: **19.03.2020**

(51) International Patent Classification (IPC):
*C07K 14/78* (2006.01)   *C12N 5/071* (2010.01)
*C12N 5/074* (2010.01)   *C12N 5/077* (2010.01)
*C12Q 1/02* (2006.01)   *G01N 33/15* (2006.01)

(52) Cooperative Patent Classification (CPC):
**C07K 14/78; C12Q 1/02; G01N 33/15**

(86) International application number:
**PCT/JP2020/012476**

(87) International publication number:
**WO 2020/203369 (08.10.2020 Gazette 2020/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **01.04.2019 JP 2019070137
21.10.2019 JP 2019191895**

(71) Applicants:
• **TOPPAN INC.
Tokyo 110-0016 (JP)**
• **Osaka University
Suita-shi, Osaka 565-0871 (JP)**

• **Kyoto Prefectural Public University Corporation
Kyoto-shi, Kyoto 602-8566 (JP)**

(72) Inventors:
• **KITANO, Shiro
Tokyo 110-0016 (JP)**
• **IRIE, Shinji
Tokyo 110-0016 (JP)**
• **MATSUSAKI, Michiya
Suita-shi, Osaka 565-0871 (JP)**
• **LOUIS, Fiona
Suita-shi, Osaka 565-0871 (JP)**
• **SOWA, Yoshihiro
Kyoto-shi, Kyoto 602-8566 (JP)**

(74) Representative: **TBK
Bavariaring 4-6
80336 München (DE)**

(54) **CELL CONSTRUCT AND CELL CONSTRUCT PRODUCTION METHOD**

(57) Disclosed is a cell structure comprising: a fragmented extracellular matrix component; and cells, wherein the cell structure comprises an intercellular vascular network, and the cells comprise at least adipocytes and vascular endothelial cells.

EP 3 950 711 A1

## Description

### Technical Field

The present invention relates to a cell structure and a method for producing a cell structure, in particular, to a cell structure comprising an intercellular vascular network and a method for producing a cell structure comprising an intercellular vascular network.

### Background Art

Known as techniques to artificially produce constructs simulating biological tissues are, for example, a method of producing a three-dimensional tissue, the method including three-dimensionally disposing cells each coated with a coating film containing collagen to form a three-dimensional tissue (Patent Literature 1), and a method of producing a three-dimensional cellular tissue, the method including: mixing cells with a cationic substance and an extracellular matrix component to give a mixture; collecting cells from the resulting mixture; and forming a cell aggregate on a substrate (Patent Literature 2). The present inventors have proposed a method for producing a large-sized three-dimensional tissue having a thickness of 1 mm or more with use of a relatively small number of cells by bringing cells and fragmented exogenous collagen into contact with each other (Patent Literature 3). Three-dimensional tissue like them are expected to be used as alternatives for experimental animals, materials for transplantation, and so on.

### Citation List

### Patent Literature

Patent Literature 1: International Publication No. WO 2015/072164
Patent Literature 2: International Publication No. WO 2017/146124
Patent Literature 3: International Publication No. WO 2018/143286

### Summary of Invention

### Technical Problem

Thick three-dimensional tissue can be produced according to any of the above-mentioned methods for producing a three-dimensional tissue. However, no method for producing adipose tissue with an intercellular vascular network formed like biological tissues has been known.
Thus, an object of the present invention is to provide a cell structure comprising an intercellular vascular network and a method for producing a cell structure comprising an intercellular vascular network.

### Solution to Problem

Specifically, the present invention relates, for example, to the followings.

[1] A cell structure comprising: a fragmented extracellular matrix component; and cells, wherein

the cell structure comprises an intercellular vascular network, and
the cells comprise at least adipocytes and vascular endothelial cells.

[2] The cell structure according to [1], wherein the vascular network is formed among the adipocytes.
[3] The cell structure according to [1] or [2], wherein the adipocytes comprise mature adipocytes.
[4] The cell structure according to any one of [1] to [3], wherein the average length of the fragmented extracellular matrix component is 100 nm or more and 400 $\mu$m or less.
[5] The cell structure according to any one of [1] to [4], wherein the content of the extracellular matrix component in the cell structure is 0.01 to 90% by mass based on the dry mass of the cell structure.
[6] The cell structure according to any one of [1] to [5], wherein the fragmented extracellular matrix component comprises collagen.
[7] The cell structure according to any one of [1] to [6], further comprising fibrin.

[8] The cell structure according to any one of [1] to [6], for transplantation.

[9] A method for producing a cell structure comprising an intercellular vascular network, the method comprising:

a contacting step of bringing a fragmented extracellular matrix component and cells into contact with each other, wherein the cells (i) comprise at least adipocytes, stem cells, and vascular endothelial cells, or (ii) comprise at least adipose stem cells and vascular endothelial cells; and

a culturing step of culturing the cells that are in contact with a fragmented extracellular matrix.

[10] The method according to [9], wherein the cells comprise adipocytes, adipose stem cells, and vascular endothelial cells.

[11] The method according to [9] or [10], wherein the adipocytes comprise mature adipocytes.

[12] The method according to any one of [9] to [11], wherein the amount of the fragmented extracellular matrix component in the contacting step is 0.1 to 100 mg per $1.0 \times 10^6$ cells.

[13] The method according to any one of [9] to [12], wherein the cell count ratio between the stem cells and the vascular endothelial cells in the contacting step is 100/1 to 1/100.

[14] The method according to any one of [9] to [13], wherein the fragmented extracellular matrix component comprises collagen.

[15] The method according to any one of [9] to [14], the method further comprising adding fibrinogen in the contacting step, or after the contacting step before the culturing step.

[16] A non-human model animal comprising the cell structure according to any one of [1] to [8] as an implant.

[17] A method for producing a non-human model animal, comprising transplanting the cell structure according to any one of [1] to [8] into a non-human animal.

[18] A method for transplanting a cell structure having vascular structure, the method comprising transplanting the cell structure according to any one of [1] to [8] into an animal.

[19] A cell structure comprising: a fragmented extracellular matrix component; and cells, wherein

the cell structure comprises an intercellular vascular network,

the cell structure is a cell aggregate formed without adhering to a support, and

the cells comprise at least adipocytes and vascular endothelial cells.

[20] The cell structure according to [19], being generally spherical.

[21] A method for producing a cell structure comprising an intercellular vascular network, the method comprising:

a contacting step of bringing a fragmented extracellular matrix component and cells into contact with each other, wherein the cells (i) comprise at least adipocytes, stem cells, and vascular endothelial cells, or (ii) comprise at least adipose stem cells and vascular endothelial cells; and

a culturing step of culturing the cells that are in contact with a fragmented extracellular matrix, wherein the culturing step comprises culturing the cells that are in contact with the fragmented extracellular matrix without the cells adhering to a support.

[22] The method according to [21], wherein the culturing step comprises separating the cells that are in contact with the fragmented extracellular matrix apart from the support.

[23] A cellular tissue comprising a plurality of the cell structures according to [19] or [20], wherein the vascular networks are connected among the plurality of the cell structures.

[24] A method for producing a cellular tissue, comprising suspension-culturing a plurality of the cell structures according to [19] or [20].

[25] A method for producing a non-human model animal, comprising transplanting a plurality of the cell structures according to [19] or [20] into a non-human animal.

[26] The method according to [25], comprising growing the cell structures for 30 days or more after transplanting into the non-human animal.

[27] The method according to [26], comprising growing the cell structures for 90 days or more after transplanting into the non-human animal.

[28] A method for evaluating an effect of a drug for inhibiting or promoting metabolism in adipose tissue, by using the cell structure according to any one of [1] to [8], [19], and [20].

[29] The method according to [28], the method comprising:

an administration step of administering the drug for inhibiting or promoting metabolism in adipose tissue to the cell structure; and

an evaluation step of evaluating the effect of the drug based on change in metabolism in the cell structure receiving administration of the drug.

[30] The method according to [29], wherein the evaluation step comprises

evaluating the effect of the drug using, as an index, change in uptake of glucose and/or fatty acid and/or change in release of glucose and/or fatty acid taken up.

[31] A method for screening for a drug for inhibiting or promoting metabolism in adipose tissue, by using the cell structure according to any one of [1] to [8], [19], and [20].

[32] The method according to [31], the method comprising:

a step of measuring metabolism in the cell structure receiving administration of the drug; and
a step of comparing metabolism in the cell structure receiving administration of the drug with metabolism in the cell structure not receiving administration of the drug, and, if the metabolism in the cell structure receiving administration of the drug is lower, selecting the drug as a candidate substance for a drug for inhibiting metabolism in adipose tissue, or comprising:

a step of measuring metabolism in the cell structure receiving administration of the drug; and
a step of comparing metabolism in the cell structure receiving administration of the drug with metabolism in the cell structure not receiving administration of the drug, and, if the metabolism in the cell structure receiving administration of the drug is higher, selecting the drug as a candidate substance for a drug to promote metabolism in adipose tissue.

[33] The method according to [32], wherein the comparison of metabolism in the cell structure is carried out using, as an index, uptake of glucose and/or fatty acid and/or release of glucose and/or fatty acid taken up.

## Advantageous Effect of Invention

According to the present invention, cell structures comprising an intercellular vascular network can be produced with ease.

## Brief Description of Drawings

Figure 1 shows photographs showing results of observation for (a) a biological tissue and (b) a cell structure in Test Example 2 with perilipin staining and CD31 staining.
Figure 2 shows a graph comparing the number of vascular branches in a cell structure in Test Example 2 with the number of vascular branches in a biological tissue.
Figure 3 shows a photograph showing a result of observation for a cell structure in Test Example 3 with perilipin staining and CD31 staining.
Figure 4 shows a photograph showing a result of observation for a cell structure in Test Example 4 with CD31 staining.
Figure 5 shows a photograph showing a result of observation for a cell structure in Test Example 5 with CD31 staining.
Figure 6 shows a photograph showing a result of observation for a cell structure in Test Example 6 with perilipin staining and CD31 staining.
Figure 7 shows a diagram illustrating the summary of Test Example 7. In the droplet in the left, circles represent mature adipocytes, open rhombuses represent ADSCs, and gray short rods represent HUVECs.
Figure 8 shows photographs showing results of fluorescence imaging for cell balls with a vascular network in Test Example 7 with Nile Red staining and CD31 staining. (b) shows a further enlarged view of one of the cell balls in (a).
Figure 9 shows photographs showing results of observation for cell balls with a vascular network with CD31 staining.
Figure 10 shows a graph showing average diameters (n = 12 cell balls/volume) of cell balls produced by using the method of Test Example 7 after culturing for 7 days.
Figure 11 shows photographs showing results of fluorescence imaging for cellular tissue produced by using a method of Test Example 8 with Nile Red staining and CD31 staining ((a) and (b) are partial enlarged photographs), and (c) a photograph showing bright-field observation of cell balls aggregating on a plate.

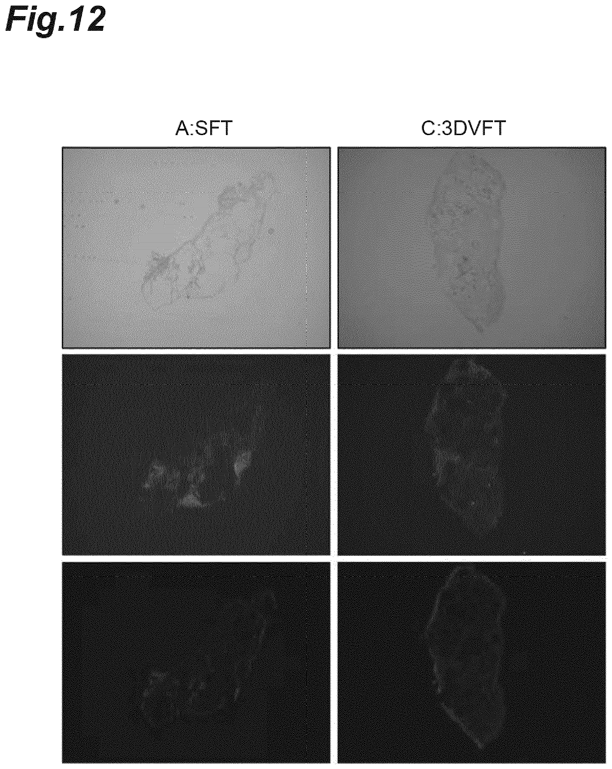

Figure 12 shows photographs showing results of fluorescence imaging for tissue collected from a transplant part after 30 days in Test Example 9 with perilipin staining and DAPI staining. A:SFT shows tissue collected from a part at which adipose tissue obtained from the femur of a human (biological tissue) through liposuction was transplanted, and C:3DVFT shows tissue collected from a part at which cell balls of (1) in Test Example 9 were transplanted. In Figure 12, the top images show results of bright-field observation, the middle images show results with perilipin staining, and the bottom images show results with DAPI staining.

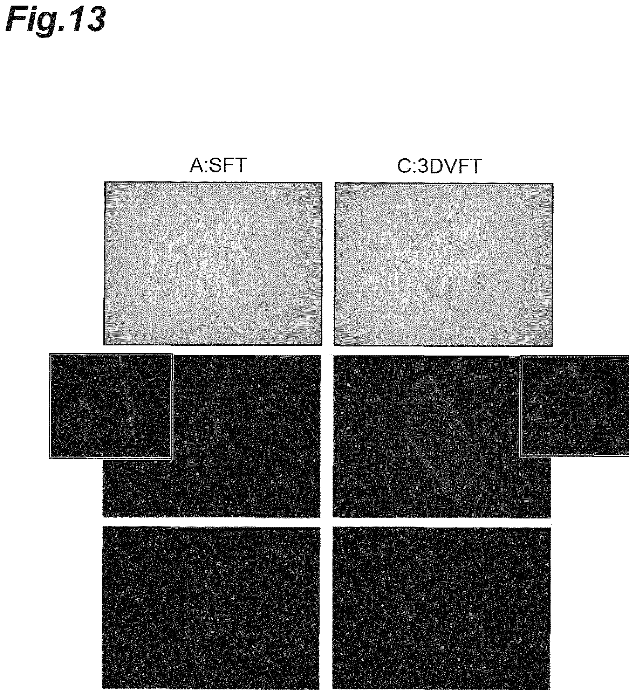

Figure 13 shows photographs showing results of fluorescence imaging for tissue collected from a transplant part after 90 days in Test Example 9 with CD31 staining and DAPI staining. A:SFT shows tissue collected from a part at which adipose tissue obtained from the femur of a human (biological tissue) through liposuction was transplanted, and C:3DVFT shows tissue collected from a part at which cell balls of (1) in Test Example 9 were transplanted. In Figure 13, the top images show results of bright-field observation, the middle images show results with CD31 staining, and the bottom images show results with DAPI staining.

Figure 14 shows photographs showing results of fluorescence imaging for a cell structure 10 minutes, 30 minutes, 60 minutes, 120 minutes, and 150 minutes after the beginning of incubation.

Figure 15 shows comparison of fluorescence intensity after 60 minutes on day 7 and day 14 after the beginning of formation for (b) cell structure including vascular endothelial cells and (a) a tissue including no vascular endothelial cell. Col I indicates that sCMF produced by using collagen I was used, and Col I+III indicates that sCMF produced by using a mixture of collagens I and III was used.

Figure 16 shows comparison of fluorescence intensity 20 minutes, 45 minutes, 70 minutes, 90 minutes, and 135 minutes after the beginning of incubation of a tissue including no vascular endothelial cell.

Figure 17 shows a graph(a) comparing fluorescence intensity (amount of glucose uptake) 20 minutes, 45 minute, 70 minutes, and 90 minutes after the beginning of incubation of a tissue including no vascular endothelial cell in Test Example 11; and a graph (b) comparing fluorescence intensity (amount of fatty acid uptake) 5 minute, 30 minutes, and 60 minutes after the beginning of incubation of a tissue including no vascular endothelial cell in Test Example 12 herein.

Figure 18 shows comparison of fluorescence intensity 0 minutes, 5 minutes, 30 minute, and 60 minutes after the beginning of incubation of (b) a cell structure including vascular endothelial cells and (a) a tissue including no vascular endothelial cell.

Figure 19 shows comparison of (a) the amount of release of glucose and (b) the amount of release of fatty acid after second incubation.

## Description of Embodiments

Hereinafter, modes for implementing the present invention will be described in detail. However, the present invention is not limited to the following embodiments.

[Cell structure]

The cell structure according to the present embodiment comprises: a fragmented extracellular matrix component; and cells comprising at least adipocytes and vascular endothelial cells, wherein the cell structure comprises an intercellular vascular network.

Artificially produced thick three-dimensional tissue are said to have difficulty in maintaining in a state without any blood vessel and need to be supplied with oxygen and so on from the external. In contrast to this, the cell structure according to the present embodiment is expected to be successfully maintained for a long period of time because an intercellular vascular network is formed like biological tissues therein. In addition, the cell structure according to the present embodiment is expected to be readily engrafted in being transplanted into mammals and so on.

Herein, the "cell structure" refers to an aggregate of cells (aggregated cell population) in which cells are three-dimensionally disposed via an extracellular matrix component, the aggregate being artificially made through cell culture. The shape of the cell structure is not limited in any way, and examples thereof include sheet-like, spherical, generally spherical, ellipsoidal, generally ellipsoidal, hemispherical, generally hemispherical, semicircular, generally semicircular, cuboidal, and generally cuboidal shapes. Here, biological tissues include sweat glands, lymphatic vessels, sebaceous glands, and so on, and their configurations are more complex than that of the cell structure. Therefore, the cell structure and biological tissues are readily distinguishable from each other. The cell structure may be a cell aggregate formed adhering to a support, or a cell aggregate formed without adhering to a support. With use of a plurality of the cell structures each being a massive aggregate without adhering to a support, cellular tissue in which vascular networks are connected among the plurality of the cell structures can be efficiently produced, as described later.

(Cells)

Herein, the "cells" are not limited in any way, and may be cells derived from a mammal such as a human, a monkey, a dog, a cat, a rabbit, a pig, a bovine, a mouse, and a rat. The site from which the cells are derived is not limited in any way, too, and the cells may be somatic cells derived, for example, from the bone, muscle, internal organ, nerve, brain, bone, skin, or blood, or germ cells. Further, the cells may be stem cells, or cultured cells such as primary cultured cells, subcultured cells, and established cells.

Herein, the "stem cells" refer to cells possessing replication competence and pluripotency. Included in stem cells are pluripotent stem cells, which possess ability to differentiate into any type of cells, and tissue stem cells (also called somatic stem cells), which possess ability to differentiate into a particular type of cells. Examples of pluripotent stem cells include embryonic stem cells (ES cells), nuclear transfer embryonic stem cells (ntES cells), and induced pluripotent stem cells (iPS cells). Examples of tissue stem cells include mesenchymal stem cells (e.g., adipose stem cells, bone marrow-derived stem cells), hematopoietic stem cells, and neural stem cells. Examples of adipose stem cells include human adipose stem cells (ADSCs).

In the cell structure according to the present embodiment, the cells comprise at least adipocytes and vascular endothelial cells.

Herein, the "adipocytes" refer to all types of adipocytes except adipose stem cells. Included in adipocytes are mature adipocytes and adipocytes not falling within adipose stem cells, it is preferable that the adipocytes comprise mature adipocytes, it is more preferable that 90% or more of the total cell count of the adipocytes be mature adipocytes, and it is even more preferable that all the adipocytes be mature adipocytes. For the adipocytes, cells collected, for example, from subcutaneous adipose tissue or epicardium-derived adipose tissue may be used, and thus-collected cells (e.g., adipose stem cells) may be induced to differentiate for use. If adipose tissue constructed from the adipocytes is to be ultimately used to simulate tissue at a particular part in the living body, it is preferable to use adipocytes derived from tissue corresponding to the tissue at the part, though the adipocytes are not limited thereto in any way.

The size of a lipid droplet can be used as an index indicating the degree of maturity of an adipocyte. The lipid droplet is an intracellular organelle that stores lipids such as triglyceride (neutral fat) and cholesterol, and has a droplet-like shape with the lipids covered by a monolayer of phospholipid. Expression of a protein unique to adipose tissue (e.g., perilipin) is found on the surface of the phospholipid. The size of the lipid droplet varies among adipocytes that have matured, and if the average value of the size of the lipid droplet is 20 μm or more, for example, the adipocytes can be regarded to have matured to some degree, that is, can be regarded as mature adipocytes.

The content of the adipocytes to the total cell count in the cell structure may be, for example, 5% or more, 10% or more, 15% or more, 20% or more, 25% or more, or 30% or more, and may be 95% or less, 90% or less, 80% or less, or 75% or less.

Herein, "vascular endothelial cells" refer to flat cells constituting the surface of the intravascular space. Examples of the vascular endothelial cells include human umbilical vein endothelial cells (HUVECs).

The content of the vascular endothelial cells to the total cell count in the cell structure may be, for example, 5% or more, 10% or more, 15% or more, 20% or more, 25% or more, or 30% or more, and may be 95% or less, 90% or less, 80% or less, or 75% or less.

In the present embodiment, the cells comprise at least adipocytes and vascular endothelial cells, and may comprise cells other than adipocytes and vascular endothelial cells. Examples of the cells other than adipocytes and vascular endothelial cells include mesenchymal cells such as fibroblasts, chondrocytes, and osteoblasts, cancer cells such as large bowel cancer cells (e.g., human large bowel cancer cells (HT29)) and liver cancer cells, cardiomyocytes, epithelial cells (e.g., human gingival epithelial cells), lymphatic endothelial cells, neurons, dendritic cells, hepatocytes, adherent cells (e.g., immunocytes), smooth muscle cells (e.g., aortic smooth muscle cells (Aorta-SMCs)), pancreatic islet cells, and keratinocytes (e.g., human epidermal keratinocytes).

The cell count ratio between the adipocytes and the vascular endothelial cells (adipocytes/vascular endothelial cells) in the cell structure according to the present embodiment is not limited in any way, and may be, for example, 100/1 to 1/100, or 50/1 to 1/50, or 20/1 to 1/1, or 10/1 to 1/1, or 8/1 to 1/1, or 7/1 to 1.2/1, or 6/1 to 1.5/1, or 5/1 to 2/1, or 3/1 to 2/1.

(Intercellular Vascular Network)

The cell structure according to the present embodiment comprises an intercellular vascular network. "Comprising an intercellular vascular network" means comprising a structure in which branched blood vessels extend among cells to surround the cells like biological tissues. Whether a vascular network like those of biological tissues is formed can be determined, for example, on the basis of the number of vascular branches and/or the vascular interbranch lengths and/or the diversity of vascular diameter in biological tissues. If the average value of the number of vascular branches in the cell structure to the average value of the number of vascular branches in biological tissues is 80% or more and 150% or less, 85% or more and 130% or less, or 90% or more and 120% or less, for example, the number of vascular branches in the cell structure may be determined to be similar to the number of vascular branches in biological tissues. If the average value of the number of vascular branches in the cell structure is 2.5 or more and 4.5 or less or 3.0 or more and 4.2 or less, for example, the number of vascular branches in the cell structure may be determined to be similar to the number of vascular branches in biological tissues. If the average value of the vascular interbranch lengths in the cell structure to the average value of the vascular interbranch lengths in biological tissues is 80% or more and 150% or less, 85% or more and 130% or less, or 90% or more and 120% or less, for example, the vascular interbranch lengths in the cell structure may be determined to be similar to the vascular interbranch lengths in biological tissues. In biological tissues, both thick blood vessels and thin blood vessels are observed. In view of this, if both blood vessels of large diameter (e.g., 10 $\mu$m or more and less than 25 $\mu$m) and blood vessels of small diameter (e.g., more than 0 $\mu$m and less than 10 $\mu$m) are observed like biological tissues, for example, determination may be made as having diversity of vascular diameter similar to that in biological tissues. If 60% or more, 70% or more, or 80% or more of all of the vascular diameters are distributed within more than 0 $\mu$m and less than 25 $\mu$m, for example, determination may be made as having diversity of vascular diameter similar to that in biological tissues. It is preferable that the cell structure according to the present embodiment comprise a vascular network among adipocytes. In this case, it is preferable, in addition to comprising a vascular network, that the adipocytes to be surrounded by the blood vessels be similar to those in biological tissues. If the average value of the size of the lipid droplet of the adipocytes in the cell structure according to the present embodiment is 20 $\mu$m to 180 $\mu$m or 100 $\mu$m to 180 $\mu$m, for example, the cell structure may be determined to include adipocytes similar to those in biological tissues. In the above comparison between biological tissues and the cell structure, biological tissues and the cell structure are compared under the same conditions (e.g., per certain specified volume, per certain specified area in the case of image analysis, per certain specified sample).

(Fragmented Extracellular Matrix Component)

Herein, the "extracellular matrix component" is an aggregate of extracellular matrix molecules formed of a plurality of extracellular matrix molecules. The extracellular matrix refers to a substance present outside of cells in organisms. Any substance can be used for the extracellular matrix as long as the substance does not adversely affect the growth of cells and the formation of a cell aggregate. Specific examples thereof include, but are not limited to, collagen, elastin, proteoglycan, fibronectin, hyaluronic acid, laminin, vitronectin, tenascin, entactin, and fibrillin. One of these may be used singly and these may be used in combination as the extracellular matrix component. The extracellular matrix component may contain, for example, a collagen component, or be a collagen component. It is preferable for the extracellular matrix component in the present embodiment to be a substance present outside of animal cells, that is, an animal extracellular matrix component. The extracellular matrix molecule may be a modified product or variant of the above-mentioned extracellular matrix molecule as long as the extracellular matrix molecule does not adversely affect the growth of cells and the formation of a cell aggregate, and may be a polypeptide such as a chemically synthesized peptide.

"Fragmenting" refers to reducing the size of an aggregate of the extracellular matrix component. The fragmented extracellular matrix component may contain a defibered extracellular matrix component. The defibered extracellular matrix component is a component obtained by defibering the above-described extracellular matrix component by application of physical force. For example, defibering is carried out under conditions that do not cleave any bond in the extracellular matrix molecule.

The method for fragmenting the extracellular matrix component such as a collagen component is not limited in any way, and fragmentation may be performed by application of physical force. The method for fragmenting the extracellular matrix component may be, for example, a method of finely crushing the extracellular matrix component in a mass. The extracellular matrix component may be fragmented with a solid phase, or fragmented in an aqueous medium. For example, the extracellular matrix component may be fragmented by application of physical force such as an ultrasonic homogenizer, a stirring homogenizer, and a high-pressure homogenizer. If a stirring homogenizer is used, the extracellular matrix component may be directly homogenized, or homogenized in an aqueous medium such as physiological saline. In addition, a millimeter-sized or nanometer-sized fragmented extracellular matrix component can be obtained by adjusting the time, rotational frequency, and so on of homogenization.

The diameter and length of the fragmented extracellular matrix component can be determined by individually analyzing the fragmented extracellular matrix component with an electron microscope.

The average length of the fragmented extracellular matrix component may be 100 nm or more and 400 $\mu$m or less, or 100 nm or more and 200 $\mu$m or less. In an embodiment, from the viewpoint of easiness in forming a thick tissue, the average length of the fragmented extracellular matrix component may be 5 $\mu$m or more and 400 $\mu$m or less, or 10 $\mu$m or more and 400 $\mu$m or less, or 100 $\mu$m or more and 400 $\mu$m or less. In another embodiment, the average length of the fragmented extracellular matrix component may be 100 $\mu$m or less, or 50 $\mu$m or less, or 30 $\mu$m or less, or 15 $\mu$m or less, or 10 $\mu$m or less, or 1 $\mu$m or less, and 100 nm or more. It is preferable that the average length of a large proportion of the fragmented extracellular matrix component to the entire fragmented extracellular matrix component be within the above numerical range. Specifically, it is preferable that the average length of 50% or more of the fragmented extracellular matrix component to the entire fragmented extracellular matrix component be within the above numerical range, and it is more preferable that the average length of 95% of the fragmented extracellular matrix component be within the above numerical range. It is preferable that the fragmented extracellular matrix component be a fragmented collagen component with the average length being in the above range.

The average diameter of the fragmented extracellular matrix component may be 50 nm to 30 $\mu$m, or 4 $\mu$m to 30 $\mu$m, or 5 $\mu$m to 30 $\mu$m. It is preferable that the fragmented extracellular matrix component be a fragmented collagen component with the average diameter being within the above range.

The average length and average diameter of the fragmented extracellular matrix component can be determined by individually measuring the fragmented extracellular matrix component with an optical microscope or the like and performing image analysis. Herein, "average length" refers to the average value of length in the longitudinal direction of a sample measured, and "average diameter" refers to the average value of length in the direction orthogonal to the longitudinal direction of a sample measured.

If the extracellular matrix component is a collagen component, the fragmented extracellular matrix component is also referred to as a "fragmented collagen component". The "fragmented collagen component" refers to a product that is obtained by fragmenting a collagen component such as a fibrous collagen component and retains the triple helix structure. It is preferable that the average length of the fragmented collagen component be 100 nm to 200 $\mu$m, it is more preferable that the average length be 22 $\mu$m to 200 $\mu$m, and it is even more preferable that the average length be 100 $\mu$m to 200 $\mu$m. It is preferable that the average diameter of the fragmented collagen component be 50 nm to 30 $\mu$m, it is more preferable that the average diameter be 4 $\mu$m to 30 $\mu$m, and it is even more preferable that the average diameter be 20 $\mu$m to 30 $\mu$m.

At least part of the fragmented extracellular matrix component may be intermolecularly or intramolecularly crosslinked. The extracellular matrix component may be intermolecularly or intramolecularly crosslinked in the extracellular matrix molecules constituting the extracellular matrix component.

Examples of the crosslinking method include, though the method is not limited in any way, physical crosslinking by application of heat, ultraviolet rays, radiation, or the like and chemical crosslinking with a crosslinking agent or by enzymatic reaction or the like. Physical crosslinking is preferred from the viewpoint that the growth of cells is not inhibited. The crosslinking (any of physical crosslinking and chemical crosslinking) may be crosslinking via covalent bonds.

If the extracellular matrix component contains a collagen component, crosslinks may be formed between collagen molecules (triple helix structures), or between collagen fine fibers formed of collagen molecules. The crosslinking may be crosslinking by heat (thermal crosslinking). Thermal crosslinking can be carried out, for example, by performing heat treatment under reduced pressure with use of a vacuum pump. If thermal crosslinking of the collagen component is performed, the extracellular matrix component may be crosslinked through formation of a peptide bond (-NH-CO-) between an amino group of a collagen molecule and a carboxy group of the same or another collagen molecule.

Alternatively, the extracellular matrix component can be crosslinked by using a crosslinking agent. The crosslinking agent may be, for example, a crosslinking agent capable of crosslinking a carboxyl group and an amino group, or a crosslinking agent capable of crosslinking amino groups. For example, aldehyde-based, carbodiimide-based, epoxide-based, and imidazole-based crosslinking agents are preferred as the crosslinking agent from the viewpoint of economic efficiency, safety, and operability, and specific examples thereof include glutaraldehyde and water-soluble carbodiimides such as 1-ethyl-3-(3-dimethylaminopropyl)carbodiimide hydrochloride and 1-cyclohexyl-3-(2-morpholinyl-4-ethyl)carbodiimide sulfonate.

Quantification of the degree of crosslinking can be appropriately selected according to the type of the extracellular matrix component, the means for crosslinking, and so on. The degree of crosslinking may be 1% or more, 2% or more, 4% or more, 8% or more, or 12% or more, and may be 30% or less, 20% or less, or 15% or less. With the degree of crosslinking being in the above range, the extracellular matrix molecules can be moderately dispersed, and redispersibility after dry storage is satisfactory.

If amino groups in the extracellular matrix component are used for crosslinking, the degree of crosslinking can be quantified on the basis of a TNBS method, for example, described in Non Patent Literature 2. The degree of crosslinking determined by the TNBS method may be in the above-mentioned range. The degree of crosslinking determined by the TNBS method is the proportion of amino groups used for crosslinking among the amino groups possessed by the extracellular matrix. If the extracellular matrix component contains a collagen component, it is preferable that the degree of crosslinking as measured by the TNBS method be within the above range.

The degree of crosslinking may be calculated by quantifying carboxyl groups. In the case of a water-insoluble extracellular matrix component, for example, quantification may be made by using a TBO (toluidine blue O) method. The degree of crosslinking determined by the TBO method may be within the above-mentioned range.

The content of the extracellular matrix component in the cell structure may be 0.01 to 90% by mass based on the cell structure (dry weight), it is preferable that the content of the extracellular matrix component be 10 to 90% by mass, it is preferable that the content of the extracellular matrix component be 10 to 80% by mass, it is preferable that the content of the extracellular matrix component be 10 to 70% by mass, it is preferable that the content of the extracellular matrix component be 10 to 60% by mass, it is preferable that the content of the extracellular matrix component be 1 to 50% by mass, it is preferable that the content of the extracellular matrix component be 10 to 50% by mass, it is more preferable that the content of the extracellular matrix component be 10 to 30% by mass, and it is more preferable that the content of the extracellular matrix component be 20 to 30% by mass.

Here, the "extracellular matrix component in the cell structure" refers to the extracellular matrix component constituting the cell structure, and may be derived from an endogenous extracellular matrix component or derived from an exogenous extracellular matrix component.

The "endogenous extracellular matrix component" refers to an extracellular matrix component produced by extracellular matrix-producing cells. Examples of extracellular matrix-producing cells include the above-mentioned mesenchymal cells such as fibroblasts, chondrocytes, and osteoblasts. The endogenous extracellular matrix component may be fibrous or nonfibrous.

The "exogenous extracellular matrix component" refers to an extracellular matrix component supplied from the external. The cell structure according to the present embodiment comprises a fragmented extracellular matrix component as an exogenous extracellular matrix component. The animal species as the origin of the exogenous extracellular matrix component may be identical to or different from the animal species as the origin of the endogenous extracellular matrix component. Examples of the animal species as the origin include humans, pigs, and bovines. The exogenous extracellular matrix component may be an artificial extracellular matrix component.

If the extracellular matrix component is a collagen component, the exogenous extracellular matrix component is also referred to as an "exogenous collagen component", and the "exogenous collagen component", referring to a collagen component supplied from the external, is an aggregate of collagen molecules formed of a plurality of collagen molecules, and specific examples thereof include fibrous collagen and nonfibrous collagen. It is preferable that the exogenous collagen component be fibrous collagen. The fibrous collagen refers to a collagen component to serve as a main component of collagen fibers, and examples thereof include collagen I, collagen II, and collagen III. A commercially available collagen may be used as the fibrous collagen, and specific examples thereof include porcine skin-derived collagen I manufactured by NH Foods Ltd. Examples of exogenous nonfibrous collagen include collagen IV.

The animal species as the origin of the exogenous extracellular matrix component may be different from the animal species as the origin of the cells. If the cells include extracellular matrix-producing cells, the animal species as the origin of the exogenous extracellular matrix component may be different from the animal species as the origin of the extracellular matrix-producing cells. In other words, the exogenous extracellular matrix component may be a xenogeneic extracellular matrix component.

Specifically, if the cell structure contains an endogenous extracellular matrix component and a fragmented extracellular matrix component, the content of the extracellular matrix component constituting the cell structure refers to the total amount of the endogenous extracellular matrix component and the fragmented extracellular matrix component. The content of the extracellular matrix can be calculated from the volume of the cell structure obtained and the mass of the cell structure after being decellularized.

If the extracellular matrix component contained in the cell structure is a collagen component, for example, examples of the method for quantifying the amount of the collagen component in the cell structure include a method of quantifying hydroxyproline as follows. Hydrochloric acid (HCl) is mixed into a lysate obtained by lysing the cell structure, incubation is performed at high temperature for a predetermined period of time and the temperature is then returned to room temperature, and the supernatant resulting from centrifugation is diluted to a predetermined concentration to prepare a sample. Hydroxyproline standard solution is treated in the same manner as for the sample, and then serially diluted to prepare standards. Each of the sample and standards is subjected to a predetermined treatment with hydroxyproline assay buffer and a detection reagent, and the absorbance at 570 nm is measured. The amount of the collagen component is calculated by comparing the absorbance of the sample and those of the standards. Alternatively, a lysate obtained by suspending the cell structure directly in hydrochloric acid of high concentration and lysing the cell structure is centrifuged to collect the supernatant, which may be used for quantification of the collagen component. The cell structure to be lysed may be as it is after recovery from the culture solution, or subjected to drying treatment after recovery and lysed with the liquid components removed. If the cell structure as it is after recovery from the culture solution is lysed for quantification of the collagen component, however, the measurement of weight of the cell structure is expected to vary because of the influence of a culture medium component absorbed by the cell structure and a culture medium residue caused by improper experimental techniques, and hence it is preferable from the viewpoint of stable measurement of the amount of the collagen component to the weight of or per unit weight of the construct to base on the weight after drying.

More specific examples of the method for quantifying the amount of the collagen component include the following method.

(Preparation of Sample)

The whole of the cell structure subjected to freeze-drying treatment is mixed with 6 mol/L HCl and incubated with a heat block at 95°C for 20 hours or more, and the temperature is then returned to room temperature. Centrifugation is performed at 13000 g for 10 minutes, and the supernatant of the sample solution is then collected. Dilution is appropriately performed with 6 mol/L HCl so that results in measurement described later fall within the range of a calibration curve, and 200 μL of the resultant is then diluted with 100 μL of ultrapure water to prepare a sample. Of the sample, 35 μL is used.

(Preparation of Standards)

To a screw cap tube, 125 μL of standard solution (1200 μg/mL in acetic acid) and 125 μL of 12 mol/l HCl are added and mixed together, and incubated with a heat block at 95°C for 20 hours, and the temperature is then returned to room temperature. Centrifugation is performed at 13000 g for 10 minutes, the supernatant is then diluted with ultrapure water to prepare 300 μg/mL S1, and S1 is serially diluted to produce S2 (200 μg/mL), S3 (100 μg/mL), S4 (50 μg/mL), S5 (25 μg/mL), S6 (12.5 μg/mL), and S7 (6.25 μg/mL). Additionally, S8 (0 μg/mL), which consists only of 90 μL of 4 mol/l HCl, is prepared.

(Assay)

The standards and the sample each in 35 μL are added to a plate (attached to a QuickZyme Total Collagen Assay kit, QuickZyme Biosciences). To each well, 75 μL of assay buffer (attached to the kit) is added. The plate is closed with a seal, and incubation is performed with shaking at room temperature for 20 minutes. The seal is removed, and 75 μL of detection reagent (reagent A:B = 30 μL:45 μL, attached to the kit) is added to each well. The plate is closed with a seal, the solutions are mixed by shaking and incubated at 60°C for 60 minutes. After sufficient cooling on ice, the seal is removed, and absorbance at 570 nm is measured. The amount of the collagen component is calculated by comparing the absorbance of the sample and those of the standards.

The collagen component occupying in the cell structure may be specified by the area ratio or volume ratio. "Specifying by the area ratio or volume ratio" refers to calculating the ratio of the existence area of the collagen component to the entire of the cell structure by any of visual observation, various microscopes, image analysis software, and so on after the collagen component in the cell structure is made distinguishable from other tissue constituents, for example, with a known staining method (e.g., immunostaining using an anti-collagen antibody, or Masson's trichrome staining). In specifying by the area ratio, which cross-section or surface in the cell structure is used to specify the area ratio is not limited, and if the cell structure is spherical, for example, specification may be made by a diagram of a cross-section passing through the generally central portion.

If the collagen component in the cell structure is specified by the area ratio, for example, the proportion of the area is 0.01 to 99% based on the total area of the cell structure, it is preferable that the proportion of the area be 1 to 99%, it is preferable that the proportion of the area be 5 to 90%, it is preferable that the proportion of the area be 7 to 90%, it is preferable that the proportion of the area be 20 to 90%, and it is more preferable that the proportion of the area be 50 to 90%. The "collagen component in the cell structure" is as described above. The proportion of the area of the collagen component constituting the cell structure refers to the proportion of the area of the total of the endogenous collagen component and exogenous collagen component. If the cell structure obtained is stained with Masson's trichrome, for example, the proportion of the area of the collagen component can be calculated as the proportion of the area of the collagen component stained blue to the total area of a cross-section passing through the generally central portion of the cell structure.

It is preferable that the remaining rate of the cell structure after trypsin treatment with a trypsin concentration of 0.25% at a temperature of 37°C and pH 7.4 for a reaction time of 15 minutes be 70% or more, it is more preferable that the remaining rate be 80% or more, and it is even more preferable that the remaining rate be 90% or more. Such a cell structure is less likely to undergo decomposition by the enzyme during or after culture, thus being stable. The remaining rate can be calculated, for example, from the mass of the cell structure before and after the trypsin treatment.

The remaining rate of the cell structure after collagenase treatment with a collagenase concentration of 0.25% at a temperature of 37°C and pH 7.4 for a reaction time of 15 minutes may be 70% or more, it is more preferable that the remaining rate be 80% or more, and it is even more preferable that the remaining rate be 90% or more. Such a cell structure is less likely to undergo decomposition by the enzyme during or after culture, thus being stable.

It is preferable that the thickness of the cell structure be 10 μm or more, it is more preferable that the thickness be 100 μm or more, and it is even more preferable that the thickness be 1000 μm or more. Such a cell structure has a more similar structure to those of biological tissues, thus being preferable as an alternative for an experimental animal and a material for transplantation. The upper limit of the thickness of the cell structure is not limited in any way, and may be, for example, 10 mm or less, or 3 mm or less, or 2 mm or less, or 1.5 mm or less, or 1 mm or less.

Here, if the cell structure is sheet-like or cuboidal, the "thickness of the cell structure" refers to the distance between the edges in the direction perpendicular to the principal plane. If unevenness is present in the principal plane, the thickness refers to the distance at the thinnest portion in the principal plane.

If the cell structure is spherical or generally spherical, the thickness refers to the diameter. Or, if the cell structure is ellipsoidal or generally ellipsoidal, the thickness refers to the minor axis. If the cell structure is generally spherical or generally ellipsoidal and unevenness is present in the surface, the thickness refers to the shortest distance among distances between two points at which a line passing through the center of gravity of the cell structure and the surface intersect.

(Fibrin)

The cell structure according to the present embodiment may contain fibrin. Fibrin is a component that is generated through the process that thrombin acts on fibrinogen to allow it to release an A chain and a B chain from the N terminus of the Aα chain and that of the Bβ chain, respectively. Fibrin is a polymer, and generally insoluble in water. Fibrin is formed by bringing fibrinogen and thrombin into contact with each other.

[Method for Producing Cell structure]

The method for producing a cell structure comprising an intercellular vascular network according to the present embodiment comprises: a contacting step of bringing a fragmented extracellular matrix component and cells into contact with each other; and a culturing step of culturing the cells that are in contact with the fragmented extracellular matrix. In the contacting step, the cells (i) comprise at least adipocytes, stem cells, and vascular endothelial cells, or (ii) comprise at least adipose stem cells and vascular endothelial cells.

(Contacting Step)

In the production method according to the present embodiment, the contacting step is a step of bringing a fragmented extracellular matrix component and cells into contact with each other.

The cells in the contacting step are (i) those comprising at least adipocytes, stem cells, and vascular endothelial cells, or (ii) those comprising at least adipose stem cells and vascular endothelial cells. The cells and each type of cells are as described above. The cells in (i) may comprise cells other than adipocytes, stem cells, and vascular endothelial cells, and the cells in (ii) may comprise cells other than adipose stem cells and vascular endothelial cells. It is preferable that the stem cells in (i) be adipose stem cells. It is preferable that the adipocytes comprise mature adipocytes.

If being dispersed in an aqueous medium, the fragmented extracellular matrix component becomes able to come into contact with the cells with ease, and the formation of the cell structure can be promoted.

In the contacting step, the extracellular matrix component and the cells contact in an aqueous medium. Examples of the contacting step include, but are not limited to, a method of mixing together an aqueous medium containing the fragmented extracellular matrix component and an aqueous medium containing the cells, a method of adding the cells to an aqueous medium containing the fragmented extracellular matrix component, a method of adding an aqueous medium containing the extracellular matrix component to a culture solution containing the cells, a method of adding the cells to an aqueous medium containing the extracellular matrix extracellular matrix component, and a method of adding the extracellular matrix component and the cells to an aqueous medium prepared in advance.

In addition, the order of bringing the cells into contact with the fragmented extracellular matrix component is not limited in any way, and, in the case of the above (i), for example, adipocytes may be added after stem cells and vascular endothelial cells are added to an aqueous medium containing the fragmented extracellular matrix component; stem cells, vascular endothelial cells, and adipocytes may be added in the presented order to an aqueous medium containing the fragmented extracellular matrix component; stem cells, vascular endothelial cells, and adipocytes may be simultaneously added to an aqueous medium containing the fragmented extracellular matrix component; and an aqueous medium containing the fragmented extracellular matrix component may be added to an aqueous medium containing stem cells, vascular endothelial cells, and adipocytes. Mixing by stirring or the like may be performed or not after each addition. In the contacting step, a step of incubating for a certain period of time may be included after the fragmented extracellular matrix component and the cells contact.

The contacting step may be carried out after a layer of cells is formed in an aqueous medium. That is, the contacting step may be carried out by forming a layer of cells in an aqueous medium and then contacting the extracellular matrix component therewith. Formation of a layer of cells before bringing into contact with the extracellular matrix component allows production of a cell structure in which the cell density of the lower layer portion is high.

The fragmented extracellular matrix component can be obtained by the above-described method. The fragmented extracellular matrix component may be obtained by fragmenting an extracellular matrix component in an aqueous medium. That is, the production method according to the present embodiment may include a step of fragmenting an extracellular matrix component in an aqueous medium (fragmenting step) before the contacting step. The aqueous medium may be the same as the above-described aqueous medium containing the fragmented extracellular matrix component.

The fragmented extracellular matrix component may be any of those exemplified above, and may contain a fragmented collagen component.

The production method according to the present embodiment may further include a step of heating an extracellular matrix component to crosslink at least part of the extracellular matrix component before the fragmenting step, or a step of heating an extracellular matrix component to crosslink at least part of the extracellular matrix component after the fragmenting step before the contacting step.

In the step of crosslinking, the temperature (heating temperature) and time (heating time) in heating the extracellular matrix component can be appropriately determined. The heating temperature may be, for example, 100°C or more, and 200°C or less, or 220°C or less. Specifically, the heating temperature may be, for example, 100°C, 110°C, 120°C, 130°C, 140°C, 150°C, 160°C, 170°C, 180°C, 190°C, 200°C, or 220°C. The heating time (time to hold at the heating temperature) can be appropriately set according to the heating temperature. In heating at 100°C to 200°C, for example, the heating time may be 6 hours or more and 72 hours or less, and is more preferably 24 hours or more and 48 hours or less. In the step of crosslinking, heating may be performed in the absence of solvent, and heating may be performed under reduced pressure.

The production method according to the present embodiment may include a drying step of drying the fragmented extracellular matrix component after the fragmenting step.

In the drying step, the defibered extracellular matrix component is dried. Drying may be carried out, for example by using a freeze-drying method. From a solution containing the fragmented extracellular matrix component and aqueous medium, the aqueous medium is removed by carrying out the drying step after the defibering step. What the aqueous medium is removed does not mean that completely no attachment of moisture is achieved in the fragmented extracellular matrix component, but means that no attachment of moisture is achieved to a degree achievable with the above-mentioned common drying technique in common sense.

The content of stem cells may be 5% or more, 10% or more, 15% or more, 20% or more, 25% or more, or 30% or more, and may be 95% or less, 90% or less, 80% or less, or 75% or less to the total cell count in the contacting step.

The content of vascular endothelial cells may be 5% or more, 10% or more, 15% or more, 20% or more, 25% or more, or 30% or more, and may be 95% or less, 90% or less, 80% or less, or 75% or less to the total cell count in the contacting step.

The concentration of the extracellular matrix component in the contacting step can be appropriately determined according to the shape and thickness of the intended cell structure, the size of a culture vessel, and so on. For example, the concentration of the extracellular matrix component in the aqueous medium in the contacting step may be 0.1 to 90% by mass, or 1 to 30% by mass.

The amount of the fragmented extracellular matrix component in the contacting step may be, per $1.0 \times 10^6$ cells, for example, 0.1 to 100 mg, 0.5 to 50 mg, 0.8 to 25 mg, 1.0 to 10 mg, 1.0 to 5.0 mg, 1.0 to 2.0 mg, or 1.0 to 1.8 mg, and may be 0.7 mg or more, 1.1 mg or more, 1.2 mg or more, 1.3 mg or more, or 1.4 mg or more, and may be 7.0 mg or less, 3.0 mg or less, 2.3 mg or less, 1.8 mg or less, 1.7 mg or less, 1.6 mg or less, or 1.5 mg or less.

It is preferable that the mass ratio between the extracellular matrix component and the cells (extracellular matrix component/cells) in the contacting step be 1/1 to 1000/1, it is more preferable that the mass ratio be 9/1 to 900/1, and it is even more preferable that the mass ratio be 10/1 to 500/1.

The cell count ratio between stem cells and vascular endothelial cells (stem cells/vascular endothelial cells) in the contacting step is not limited in any way, and may be, for example, 100/1 to 1/100, or 50/1 to 1/50, or 20/1 to 1/1, or 10/1 to 1/1, or 8/1 to 1/1, or 7/1 to 1.2/1, or 6/1 to 1.5/1, or 5/1 to 2/1, or 3/1 to 2/1.

Adding fibrinogen and/or thrombin may be included in the contacting step, or after the contacting step before the culturing step. If both fibrinogen and thrombin are added, for example, fibrinogen and thrombin may be simultaneously added, and thrombin may be added after fibrinogen has been added. The timing to add fibrinogen and/or thrombin is not limited in any way, and, for example, fibrinogen and/or thrombin may be added to an aqueous medium containing stem cells, vascular endothelial cells, adipocytes, and the extracellular matrix component, or added to an aqueous medium containing stem cells, vascular endothelial cells, and the extracellular matrix component. Alternatively, for example, adipocytes and then thrombin may be added after fibrinogen is added to an aqueous medium containing stem cells, vascular endothelial cells, and the extracellular matrix component. With addition of fibrinogen and/or thrombin, it can become easier to suppress shrink that can occur in the culturing step described later and control the shape and size of the cell structure. In addition, a suspension of the cells and the extracellular matrix component can be gelled, and hence peeling-off from a culture vessel (support) can be facilitated after the suspension is dropped on the culture vessel. If the cells comprise adipocytes that have matured, the adipocytes may float by the influence of the lipid droplets inside the adipocytes that have matured and be disadvantageously cultured in a state in which the adipocytes and other cells are inhomogeneously present; however, the gelation of the suspension retains the state in which the cells and the extracellular matrix component are homogeneously mixed, and makes it easier to retain a state in which the cells and the extracellular matrix component are in close proximity to each other.

A step of precipitating both the extracellular matrix component and the cells in the aqueous medium may be further included after the contacting step before the culturing step. The distribution of the extracellular matrix component and the cells in the cell structure becomes more homogeneous by carrying out such a step. The specific method is not limited in any way, and examples thereof include a method of performing a centrifugal operation for a culture solution containing the extracellular matrix component and the cells.

(Culturing Step)

In the production method according to the present embodiment, the culturing step is a step of culturing the cells that are in contact with the fragmented extracellular matrix.

The method for culturing the cells that are in contact with the fragmented extracellular matrix is not limited in any way, and any preferred culture method can be applied according to the type of the cells to be cultured. For example, the culture temperature may be 20°C to 40°C, or 30°C to 37°C. The pH of the culture medium may be 6 to 8, or 7.2 to 7.4. The culture time may be 1 day to 2 weeks, or 1 week to 2 weeks.

The culture vessel (support) to be used for culture of the cells that are in contact with the fragmented extracellular matrix is not limited in any way, and may be, for example, a well insert, a low attachment plate, or a plate, for example, having a U-shaped or V-shaped bottom. The cells may be cultured with adhering to a support, the cells may be cultured without adhering to a support, and the cells may be cultured with the cells separated from a support at the middle of culturing. If the cells are cultured without adhering to a support or cultured with the cells separated from a support at the middle of culturing, it is preferable to use a plate, for example, having a U-shaped or V-shaped bottom or a low attachment plate, which inhibits the cells from adhering to a support.

The culture medium is not limited in any way, and a preferred culture medium can be selected according to the type of the cells to be cultured. Examples of the culture medium include the culture media Eagle's MEM, DMEM, Modified Eagle Medium (MEM), Minimum Essential Medium, RPMI, and GlutaMax Medium. The culture medium may be a culture medium supplemented with serum or serum-free culture medium. The culture medium may be a mixed culture medium obtained by mixing two culture media.

The cell density in the culture medium in the culturing step can be appropriately determined according to the shape and thickness of the intended cell structure, the size of the culture vessel, and so on. For example, the cell density in the culture medium in the culturing step may be 1 to $10^8$ cells/mL, or $10^3$ to $10^7$ cells/mL. The cell density in the culture medium in the culturing step may be the same as the cell density in the aqueous medium in the contacting step.

It is preferable that the shrink rate during culturing of the cell structure produced with the production method according to the present embodiment be 20% or less, it is more preferable that the shrink rate be 15% or less, and it is even more preferable that the shrink rate be 10% or less. The shrink rate can be calculated, for example, with the following expression. In the expression, L1 denotes the length of the longest part of the cell structure on day 1 after the beginning of culturing, and L3 denotes the length of the corresponding part of the cell structure on day 3 after the beginning of culturing.

$$\text{Shrink rate (\%)} = \{(L1-L3)/L1\} \times 100$$

Although the shrink rate is calculated from the cell structure on day 1 after the beginning of culturing and the cell structure on day 3 after the beginning of culturing, calculation may be made from the cell structure at any time point of culture period including the time point of the end of culturing. For example, calculation may be made from the cell structure on day 1 after the beginning of culturing and the cell structure on day 2 after the beginning of culturing, calculation may be made from the cell structure on day 1 after the beginning of culturing and the cell structure on day 5 after the beginning of culturing, and calculation may be made from the cell structure on day 1 after the beginning of culturing and the cell structure on day 8 after the beginning of culturing.

After the above culturing step (hereinafter, also referred to as the "first culturing step"; the contacting step in the first is also referred to as the "first contacting step"), a step of contacting cells (second contacting step) and a step of culturing cells (second culturing step) may be further included. The cells in the second contacting step and second culturing step may be of the same type as or different type from that of the cells used in the first contacting step and first culturing step. Through the second contacting step and second culturing step, a cell structure of bilayer structure can be produced. Further, with repeated inclusion of a contacting step and a culturing step, a cell structure with a plurality of layers can be produced, and thus more complex tissues similar to those in the living body can be produced.

According to the production method according to the present embodiment, a cell structure comprising an intercellular vascular network can be produced. The cell structure comprising an intercellular vascular network is as described above.

The above culturing step may include culturing the cells that are in contact with the fragmented extracellular matrix without adhering to a support. Thereby, a cell structure which is a cell aggregate formed without adhering to a support can be produced. If the cells that are in contact with the fragmented extracellular matrix is adhering to a support, the above culturing step may include separating the cells that are in contact with the fragmented extracellular matrix apart from the support. If the cells that are in contact with the fragmented extracellular matrix are not adhering to a support from the beginning in the culturing step, a cell structure which is a cell aggregate formed without adhering to a support can be produced by culturing as it is.

The method for separating the cells that are in contact with the fragmented extracellular matrix apart from a support is not limited in any way, and, for example, the cells may be separated from a support through use of a low attachment support and addition of a culture solution, the cells may be physically separated from a support, for example, by using an instrument in a direct manner, the cells may be separated from a support by application of vibration, and the cells may be separated from a support by applying stimulus with use of a support onto the surface of which a functional material that unbinds the support and cells in response to stimulus such as heat and light has been applied. If the cells are separated from a support by addition of a culture solution, any of the culture media and so on exemplified above can be used for the culture solution.

Examples of the method for culturing the cells that are in contact with the fragmented extracellular matrix without adhering to a support from the beginning in the culturing step, include a method in which a suspension containing the cells that are in contact with the fragmented extracellular matrix is gelled and added dropwise into a culture solution for culturing, and a method in which the shapes of the cells that are in contact with the fragmented extracellular matrix are fixed to some extent in a solvent of high viscosity and then returned into a culture vessel with the solvent exclusively removed.

In the above culturing step, the timing to separate the cells that are in contact with the fragmented extracellular matrix apart from a support is not limited in any way, and separating may be carried out, for example, 1 day to 7 days after the beginning of culturing, or 1 hour to 24 hours after the beginning of culturing, or 1 minute to 60 minutes after the beginning of culturing, or 5 minutes to 30 minutes after the beginning of culturing, or 10 minutes to 20 minutes after the beginning of culturing.

The culture period after separating the cells that are in contact with the fragmented extracellular matrix apart from a support is not limited in any way, and may be 1 day or more, or 1 day to 21 days, or 3 days to 14 days, or 7 days to 14 days.

[Cellular Tissue and Production Method Therefor]

With use of a plurality of the above-described cell structures each being a cell aggregate formed without adhering to a support, that is, "cell structures each comprising a fragmented extracellular matrix component and cells, wherein each cell structure comprises an intercellular vascular network and is a cell aggregate formed without adhering to a support, and the cells comprise at least adipocytes and vascular endothelial cells", cellular tissue in which the vascular networks are connected among the plurality of the cell structures can be produced.

Production of the cellular tissue includes suspension-culturing the plurality of the cell structures without adhering to a support. The plurality of the cell structures adheres to each other in the course of suspension culture and the vascular networks are connected among the cell structures, and hence a large cellular tissue with vascular networks joined together can be produced with ease. The number of the cell structures and size thereof according to the application of the cellular tissue, the desired size of the cellular tissue, and so on can be appropriately selected. In addition, selection can be appropriately made for the type of the culture medium (culture solution) for use in the suspension culture and culture conditions, and, for example, any of the culture media and conditions as exemplified in the above section (Culturing Step) can be applied.

[Applications of Cell structure]

The cell structure according to the present embodiment, in which an intercellular vascular network is formed like biological tissues as described above, is expected to be readily engrafted in being transplanted into mammals and so on, and hence applicable to transplantation. One cell structure may be used for transplantation, and a plurality of cell structures may be used for transplantation. In the case of a plurality of cell structures, for example, 1 to 1000, 10 to 500, or 50 to 200 cell structures can be used.

The animal as a subject of transplantation is not limited in any way, and may be, for example, a mammal, or a human, or a non-human animal such as a monkey, a dog, a cat, a rabbit, a pig, a bovine, a mouse, and a rat.

The transplantation method according to the present embodiment includes transplanting the cell structure having vascular structure according to the present embodiment into an animal. Before the transplanting, preparing an animal to be subjected to transplantation and/or producing the cell structure by using the above-described method may be further included. The transplantation method is not limited in any way, and a known surgical method or the like can be appropriately applied according to the subject of transplantation. Examples of the surgical method include incising the skin of a subject of transplantation and subcutaneously transplanting in a direct manner; and subcutaneously injecting into a subject of transplantation with a syringe or the like. One cell structure may be transplanted, a plurality of cell structures may be transplanted, and a cellular tissue including a plurality of cell structures may be transplanted. A cell structure or cellular tissue collected from the inside of a culture medium may be used, a cell structure or cellular tissue, for example, appropriately gelled or semi-gelled (e.g., fibrin gel) according to the form of transplantation may be used, and a dispersion in which a plurality of cell structures is dispersed may be used. Alternatively, a product obtained by adding fibrin to a plurality of cell structures, collected from the inside of a culture medium, being a cell aggregate formed without adhering to a support may be used for transplantation. The cell structure containing adipocytes according to the present embodiment can be applied, for example, to tissue reconstruction for aftercare of traumas, soft tissue defects caused by tumor resection, mastectomy, and so on.

The vascular network of the transplanted cell structure is connected to the blood vessel of the subject of transplantation itself around the transplant site of the subject of transplantation. If a plurality of cell structures each being a cell aggregate formed without adhering to a support is used, the vascular networks are connected also among the plurality of cell structures. Adipose tissue formed in the subject of transplantation by transplanting a plurality of cell structures being a cell aggregate formed without adhering to a support is much superior in fixability because the vascular networks are connected among the plurality of cell structures and in addition connected to the blood vessel of the subject of transplantation itself.

The method for producing a non-human model animal according to the present embodiment comprises transplanting the cell structure according to the present embodiment into a non-human animal. Before the transplanting, preparing a non-human animal to be subjected to transplantation and/or producing the cell structure by using the above-described method may be further included. The transplantation method is as described above, one cell structure may be transplanted, a plurality of cell structures may be transplanted, and a cellular tissue including a plurality of cell structures may be transplanted. The method for producing a non-human model animal according to the present embodiment may include, after transplanting the cell structure into a non-human animal, growing, for example, for 7 days or more, 30 days or more, or 90 days or more. The non-human model animal according to the present embodiment can be used to associate data obtained with animal experiment to human cases. In order to produce the non-human model animal, it is preferable to use a non-human animal whose rejection to implants (grafts) has been suppressed, for example, an immunodepleted or immunodeficient non-human animal. The non-human model animal can be used as a pathological in vitro model for inflammatory diseases and so on related to adipose tissue, and can be used for screening for pharmaceuticals against diabetes mellitus, obesity, or the like and for assay screening for cosmetics against cellulite, obesity, or the like.

The cell structure according to the present embodiment itself can also be applied as an alternative for an experimental animal, a material for transplantation, and so on, and, in a specific example, can be applied to tissue reconstruction, a pathological in vitro model, screening for pharmaceuticals (evaluation of drugs), assay screening for cosmetics, and so on, as described above.


[Method for Evaluating Effect of Drug and Screening Method]


As described above, the cell structure according to the present embodiment has a structure, like biological tissues, in which branched blood vessels extend among cells in such a way as to surround cells. Therefore, evaluation of an effect of a drug for inhibiting or promoting metabolism in adipose tissue and screening for a drug can be carried out by using the cell structure according to the present embodiment.

Provided as an embodiment of the present invention is a method for evaluating an effect of a drug for inhibiting or promoting metabolism in adipose tissue by using the cell structure, the method comprising: an administration step of administering the drug for inhibiting or promoting metabolism in adipose tissue to the cell structure; and an evaluation step of evaluating the effect of the drug based on change in metabolism in the cell structure receiving administration of the drug. According to the present embodiment, the effect of a drug for inhibiting or promoting metabolism in adipose tissue can be effectively evaluated.

In the administration step, a drug for inhibiting or promoting metabolism in adipose tissue is administered to the cell structure. The drug may be a drug known to inhibit or promote metabolism in adipose tissue, or a drug not known to inhibit or promote metabolism in adipose tissue.

Examples of drugs to inhibit metabolism in adipose tissue include insulin and TNF$\alpha$, inhibitors. Insulin is known to promote uptake of fatty acid and glucose. It is known that adipocytes take up fatty acid primarily via the transporters FATP-1 and FATP-4 in the living body, and TNF$\alpha$, inhibits the functions of these transporters to suppress uptake of fatty acid. Examples of drugs to promote metabolism in adipose tissue include apigenin, cytochalasin B, and isoproterenol. It is known that adipocytes take up glucose primarily via the transporters GLUT-1 and GLUT-4 in the living body, and apigenin and cytochalasin B inhibit the functions of these transporters to suppress uptake of glucose. It is known that catecholamine promotes release of fatty acid and glucose taken up, and hence isoproterenol, which is artificially synthesized catecholamine, functions as a release promoter for fatty acid and glucose. The drug for inhibiting or promoting metabolism in adipose tissue may be an uptake promoter or inhibitor for fatty acid and/or glucose or release promoter or inhibitor for fatty acid and/or glucose other than the above substances.

Administration of the drug for inhibiting or promoting metabolism in adipose tissue may be carried out by using a culture medium containing the drug as a culture medium for culture of the cell structure, or by adding the drug to a culture medium for culture of the cell structure.

The cell structure to which the drug for inhibiting or promoting metabolism in adipose tissue is to be administered may consist of a cell structure cultured for 1 day or more, or consist of a cell structure cultured for 5 days or more, or consist of a cell structure cultured for 6 days or more, or consist of a cell structure cultured for 7 days or more, or consist of a cell structure cultured for more than 7 days, or consist of a cell structure cultured for a period of 7 days or more and 14 days or less.

In the evaluation step, the drug efficacy is evaluated based on change in metabolism in the cell structure receiving administration of the drug. The drug efficacy can be evaluated using, as an index, metabolism in the cell structure, for example, change in uptake of glucose and/or fatty acid and/or change in release of glucose and/or fatty acid taken up. Other metabolic systems in adipose tissue can be similarly evaluated using, as an index, change in metabolites other than glucose and fatty acid in adipose tissue. Change in metabolism in the cell structure may be change in the amount of uptake of glucose and/or fatty acid per unit time in the cell structure and/or change in the amount of release of glucose and/or fatty acid per unit time in the cell structure. The drug efficacy may be evaluated on the basis of only one change of the above indexes, and may be evaluated on the basis of two or more of the above indexes. Change in metabolism may be evaluated by qualitative comparison, and may be evaluated by quantitative comparison.

The evaluation step can be carried out, for example, by comparing metabolism in the cell structure receiving administration of the drug with metabolism in the cell structure not receiving administration of the drug.

The evaluation step may be carried out a plurality of times. Specifically, evaluation of the drug efficacy may be carried out a plurality of times at predetermined intervals after administration of the drug.

In the evaluation step, for example, if the metabolism in the cell structure receiving administration of the drug is lower than the metabolism in the cell structure not receiving administration of the drug, the drug may be evaluated to be effective as a drug for inhibiting metabolism in adipose tissue; and if the metabolism in the cell structure receiving administration of the drug is higher or no change is found, the drug may be evaluated to be ineffective as a drug for inhibiting metabolism in adipose tissue. If the metabolism in the cell structure receiving administration of the drug is higher than the metabolism in the cell structure not receiving administration of the drug, the drug may be evaluated to be effective as a drug to promote metabolism in adipose tissue; and if the metabolism in the cell structure receiving administration of the drug is lower or no change is found, the drug may be evaluated to be ineffective as a drug to promote metabolism in adipose tissue.

Provides as an embodiment of the present invention is a method for screening for a drug for inhibiting or promoting metabolism in adipose tissue by using the cell structure. According to the present embodiment, a drug for inhibiting or promoting metabolism in adipose tissue can be effectively selected.

The method for screening for a drug for inhibiting or promoting metabolism in adipose tissue by using the cell structure may include a step of selecting a drug evaluated to be effective as a drug for inhibiting or promoting metabolism in adipose tissue in the evaluation step in the above evaluation method.

For example, the screening method can comprise:

a step of measuring metabolism in the cell structure receiving administration of the drug; and
a step of comparing metabolism in the cell structure receiving administration of the drug with metabolism in the cell structure not receiving administration of the drug, and, if the metabolism in the cell structure receiving administration of the drug is lower, selecting the drug as a candidate substance for a drug for inhibiting metabolism in adipose tissue, or comprise:

a step of measuring metabolism in the cell structure receiving administration of the drug; and
a step of comparing metabolism in the cell structure receiving administration of the drug with metabolism in the cell structure not receiving administration of the drug, and, if the metabolism in the cell structure receiving administration of the drug is higher, selecting the drug as a candidate substance for a drug to promote metabolism in adipose tissue.

The comparison of metabolism in the cell structure can be carried out, for example, using, as an index, uptake of glucose and/or fatty acid and/or release of glucose and/or fatty acid taken up. Change in metabolites other than glucose and fatty acid in adipose tissue may be used as an index. The comparison of metabolism in the cell structure may be carried out, for example, using, as an index, the amount of uptake of glucose and/or fatty acid per unit time in the cell structure and/or the amount of release of glucose and/or fatty acid per unit time in the cell structure. Metabolism in the cell structure may be compared on the basis of only one of the above indexes, and may be compared on the basis of two or more of the above indexes. The comparison may be qualitative comparison or quantitative comparison.

The candidate substance may be, for example, an uptake promoter or inhibitor for fatty acid and/or glucose or release promoter or inhibitor for fatty acid and/or glucose, or an uptake promoter or uptake inhibitor or release promoter or release inhibitor for a metabolite other than glucose and fatty acid.

The comparison of metabolism in the cell structure may be carried out a plurality of times. Specifically, the comparison of metabolism in the cell structure may be carried out a plurality of times at predetermined intervals after administration of the drug.

Administration of a drug for inhibiting or promoting metabolism in adipose tissue can be carried out in the same manner as in the above-described evaluation method. For the cell structure and so on for use, those described above can be used, similarly.

Examples

<Test Example 1: Production of Defibered Collagen Component>

By heating 100 mg of a sponge fragment of porcine skin-derived collagen I (manufactured by NH Foods Ltd.) at 200°C for 24 hours, a collagen component at least part of which was crosslinked (crosslinked collagen component) was obtained. No large change in appearance was found in collagen after the heating at 200°C. Into a 15-mL tube, 50 mg of the crosslinked collagen component was put, to which 5 mL of ultrapure water was added, and homogenization was performed by using a homogenizer (AS ONE Corporation VH-10) for 6 minutes to defiber the crosslinked collagen component.

Centrifugation was performed at 10000 rpm under 21°C for 10 minutes. The supernatant was sucked, and the collagen pellet was mixed with 5 mL of fresh ultrapure water to produce a collagen solution. An operation in which the tube containing the collagen solution with being kept on ice was ultrasonicated with a sonicator (Sonics and Materials, Inc. VC50) at 100 V for 20 seconds, the sonicator was removed, and the tube containing the collagen solution was cooled on ice for 10 seconds was repeated 100 times, and thereafter the collagen solution was filtered through a filter of 40 $\mu$m in pore size to afford a dispersion containing a defibered collagen component (sCMF). The dispersion was freeze-dried by using a conventional method to afford a defibered collagen component (sCMF) as a dried product. The average length (length) of sCMF was 14.8 $\pm$ 8.2 $\mu$m (N = 20).

<Test Example 2: Production and Evaluation of Cell Structure (1)>

Cells, reagents, a production method, and so on used for production of a cell structure are as follows.

(Cells and Collagen)

- Human adipose tissue (femur-derived) to obtain primary human mature adipocytes and human adipose stem cells (ADSCs) (provided by University Hospital Kyoto Prefectural University of Medicine)
- Human umbilical vein endothelial cells (HUVECs) (#C-2517A manufactured by Lonza)
- Defibered collagen component (sCMF) (produced in Test Example 1) [0119] (Reagents)
- Insulin from bovine pancreas (Sigma-Aldrich Co. LLC #I1882)
- Freeze-dried powder of thrombin from bovine plasma (Sigma-Aldrich Co. LLC #T4648)
- Collagenase from Clostridium histolyticum Type I (Sigma-Aldrich Co. LLC #C0130)
- Fibrinogen from bovine plasma Type I-S (Sigma-Aldrich Co. LLC #F8630)
- DMEM (high-glucose, NACALAI TESQUE, INC.)
- EGM-2MV BulletKit with growth factors (#C-2517A manufactured by Lonza)

(Culture Medium and Solutions)

- EGM-2 culture medium: obtained by mixing 500 mL of EBM-2 and EGM-2 supplement growth factors and storing at 4°C
- 10 mg/mL insulin stock solution: obtained by dissolving 100 mg of the above insulin from bovine pancreas in 10 mL of 1% glacial acetic acid diluted with water (pH $\leq$ 2), aliquoting the resultant into equal amounts in Eppendorf tubes, and storing at -20°C
- 2 mg/mL collagenase solution: 2.5 g of BSA was mixed in advance with 50 mL of DMEM (0% FBS, 1% antibiotic). To digest all adipose tissues in a 6-well plate, 26 mg of collagenase Type I was mixed in 13 mL of DMEM (0% FBS, 5% BSA, 1% antibiotic), and the resultant was filtered through a filter of 0.2 $\mu$m in pore size for use.
- 50 mg/mL fibrinogen stock solution: 50 mg of fibrinogen was weighed in an Eppendorf tube, and 1 mL of DMEM (0% FBS, 1% antibiotic) was immediately added thereto. After the tube was shaken by hand for mixing, the tube was placed in a water bath at 37°C for 3 to 5 minutes, and the resultant was filtered through a filter of 0.2 $\mu$m in pore size and aliquoted into equal amounts in Eppendorf tubes for use.

- 202 U/mL thrombin stock solution: 202 U of thrombin was weighed in an Eppendorf tube, 1 mL of DMEM (0% FBS, 1% antibiotic) was immediately added thereto, and the tube was placed in a water bath at 37°C for 3 to 5 minutes for dissolution. Thereafter, the resultant was filtered through a filter of 0.2 $\mu$m in pore size and aliquoted into equal amounts in Eppendorf tubes for use.

(Production Method)

A fragment of human adipose tissue was washed with PBS containing 5% antibiotic. Into six wells in a 6-well plate, 4 to 6 g of the tissue was distributed. In 2 mL of 2 mg/mL collagenase solution, the tissues were finely cut into pieces of about 1 to 3 mm in size by using scissors and tweezers. After incubation at 37°C and 230 rpm for 1 hour, mixing was performed with a 10-mL pipette for 30 minutes. Each lysate was filtered through an iron mesh of 500 $\mu$m in pore size, and 2 mL of DMEM was added to each well to recover all the cells digested, which was then centrifuged at 200 g under room temperature (15 to 25°C) for 3 minutes. Mature adipocytes are contained in the upper layer, a yellow oily layer, and adipose stem cells and hemocytes are contained in the pellet. The medium between the upper layer and the lower layer was sucked and discarded by using a long needle and a 10-mL syringe, and the mature adipocytes contained in the upper layer and the adipose stem cells and hemocytes contained in the lower layer were each washed twice with 25 mL of PBS (5% BSA, 1% antibiotic). In the washing, centrifugation was performed in the same manner as described above to separate into three layers of an upper layer, a lower layer, and a medium between the upper layer and the lower layer, and the medium between the upper layer and the lower layer was sucked and discarded. After washing was performed twice, washing was performed with 25 mL of DMEM.

Only the upper layer containing mature adipocytes was collected, and aliquoted in Eppendorf tubes. The nuclei were stained by Hoechst staining (1000-fold diluted Hoechst, staining for 15 minutes), and the cell count was determined on a Turker Burk hemocytometer through a fluorescence microscope.

The pellet containing ADSCs was suspended in 10 mL of DMEM, seeded in a 10-cm dish, and subcultured. The ADSCs were separated from the dish by using trypsin/EDTA and suspended in 1 mL of DMEM, and the cell count was determined.

HUVECs purchased from Lonza were suspended in 10 mL of DMEM, seeded in a 10-cm dish, and subcultured. The HUVECs were separated from the dish by using trypsin/EDTA and suspended in 1 mL of DMEM, and the cell count was determined.

Weighed was 1 mg of sCMF, to which 100 $\mu$L of DMEM was added, and the resultant was gently mixed until a situation that only small particles of sCMF were observed was achieved. Centrifugation was performed at 10000 rpm under room temperature for 1 minute, and the supernatant was sucked to afford an sCMF pellet. On the sCMF pellet, 250000 cells of ADSCs and 125000 cells of HUVECs (ADSCs:HUVECs = 2:1) were gently added, centrifugation was performed, without mixing, at 3500 rpm under room temperature for 1 minute, and the supernatant was sucked. Thereto, 0.3 mg of fibrinogen (6 $\mu$L of 50 mg/mL fibrinogen stock solution) was added, and gently mixed with the cells and sCMF. Further, 300000 cells of mature adipocytes were added and gently mixed. A small volume of DMEM was added, as necessary, to adjust the total volume to 70 $\mu$L. Immediately, 0.15 U thrombin (0.71 $\mu$L of 202 U/mL thrombin stock solution) was added and mixed, and then the mixture was slowly seeded on a Transwell placed on a 6-well adapter on a 6-well plate.

Incubation was performed in an incubator at 37°C for 1 hour to cause gelation, and 12 mL of EGM-2 culture medium containing insulin at a final concentration of 10 $\mu$g/mL was added. The culture medium in 12 mL was replaced every 2 to 3 days until day 7 after the beginning of culturing.

Fluorescence imaging for the cell structure was carried out with the following procedure. The Transwell containing the cell structure obtained was transferred to a 24-well plate. After washing with 2 mL of PBS, the tissue was fixed by using 2 mL of 4% PFA under 4°C overnight. Washing was performed three times with 2 mL of PBS. The cells were permeabilized with 0.05% Triton/PBS (500 $\mu$L to the inside of the Transwell, 500 $\mu$L to the outside of the Transwell) under room temperature for 7 minutes, and washed three times with 2 mL of PBS.

Blocking was performed with 1% BSA/PBS solution (500 $\mu$L to the inside of the Transwell, 500 $\mu$L to the outside of the Transwell) under room temperature for 1 hour. The BSA solution was sucked, and 100 $\mu$L of primary antibody solution (CD31 and perilipin 100-fold diluted with 1% BSA/PBS solution) was added (50 $\mu$L to the inside of the Transwell, 50 $\mu$L to the outside of the Transwell). A wet wipe was laid beneath the plate, and the plate was covered with an aluminum foil and incubated under 4°C overnight. After washing was performed three times with 2 mL of PBS, 100 $\mu$L of secondary antibody solution (AlexaFluor647-labeled anti-mouse anti-CD31 antibody and AlexaFluor488-labeled anti-rabbit anti-perilipin antibody 200-fold diluted with 1% BSA/PBS solution, 1000-fold diluted Hoechst) was added (50 $\mu$L to the inside of the Transwell, 50 $\mu$L to the outside of the Transwell). A wet wipe was laid beneath the plate, and the plate was covered with an aluminum foil and incubated under room temperature for 2 hours, and then washed four times with 2 mL of PBS.

The membrane of the Transwell was cut, the gel was directly disposed on the bottom of a glass-bottom dish containing a small volume of PBS, and the stained cell structure was observed by using a confocal laser scanning microscope (FV3000, manufactured by Olympus Corporation) with laser excitation lights at 640 nm (AlexaFluor647) and 488 nm (AlexaFluor488).

The diameters of lipid droplets in the cell structure produced were measured by using an electron microscope. The vascular diameters and vascular interbranch lengths were measured with Image J.

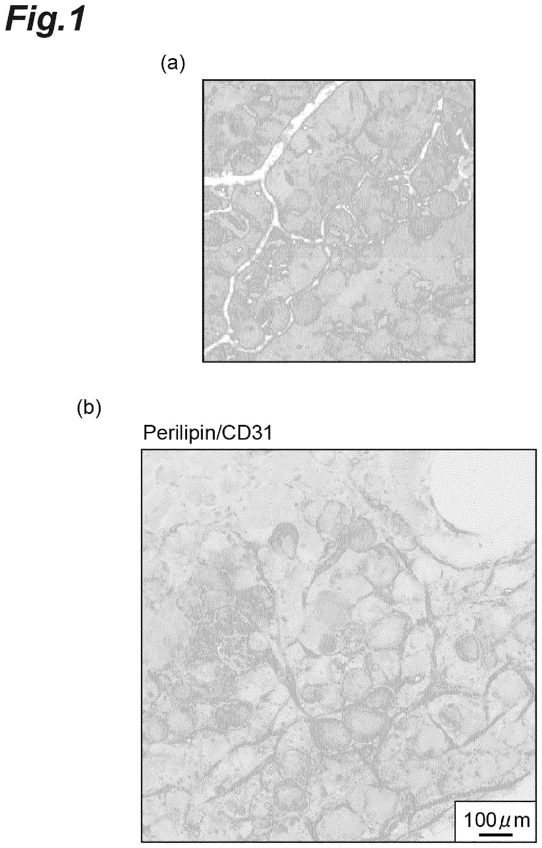

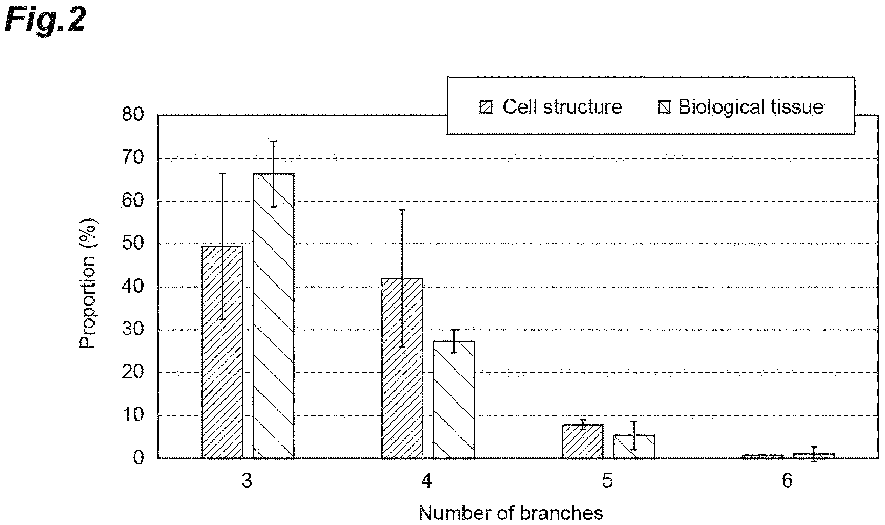

Figure 1 shows the result of fluorescence imaging (20× magnification) for the cell structure with a vascular network. (a) shows a biological tissue obtained by directly fixing adipose tissue collected from a living body, and (b) shows the cell structure. It was confirmed that, like the biological tissue, a vascular network was formed in such a way to surround mature adipocytes (large, round, eye-shaped lipid droplets) in the cell structure. Such a vascular network was formed even in the inside of the cell structure. The average value of the diameters of lipid droplets was 85 $\mu$m (N = 50), which was close to the average value of the diameters of mature adipocytes in the biological tissue (72 $\mu$m (N = 50)). The blood vessels formed were hollow like those of the biological tissue, and both those of large diameter (e.g., 10 $\mu$m or more and less than 25 $\mu$m) and those of small diameter (e.g., more than 0 $\mu$m and less than 10 $\mu$m) were observed as with the case of the biological tissue. As demonstrated in Figure 2, it was found that the number of vascular branches was also close to the number thereof in the biological tissue. In addition, it was found that the distribution of vascular interbranch lengths was 0 to 100 $\mu$m: 42.5%, 100 to 200 $\mu$m: 39.4%, more than 200 $\mu$m: 18.1% (N = 180), which was close to the distribution of vascular interbranch lengths of a vascular network in the biological tissue (0 to 100 $\mu$m: 61.8%, 100 to 200 $\mu$m: 24.7%, more than 200 $\mu$m: 13.5% (N = 180)). In Test Example 2, vascular interbranch lengths of 50 $\mu$m to 100 $\mu$m account for a large proportion, and these values are close to the sizes of mature adipocytes. As explained, for example, in J. Silha et al., "Angiogenic factors are elevated in overweight and obese individuals", International Journal of Obesity (2005) 29, 1308-1314, it is known that in the living body, blood vessels are formed among adipocytes with the blood vessels surrounding individual adipocytes. The result that the vascular interbranch lengths were distributed as shown above suggested the possibility that the cell structure in Test Example 2 succeeded in accurately simulating actual adipose tissue in the living body.

<Test Example 3: Production and Evaluation of Cell Structure (2)>

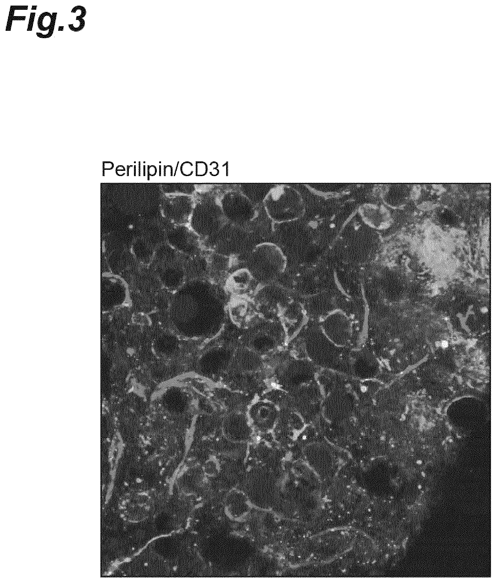

An attempt was made to produce a cell structure with the same method as in Test Example 2, except that 500000 cells of mature adipocytes, 2 mg of sCMF, and 0.6 mg of fibrinogen were used, and as a result a cell structure in which a vascular network was formed in such a way to surround mature adipocytes was successfully produced. Figure 3 shows the result of fluorescence imaging (10× magnification) for the cell structure stained in the same manner as in Test Example 2. It was demonstrated that a cell structure with a vascular network can be produced even if the cell count of mature adipocytes and the amount of sCMF are changed.

<Test Example 4: Production and Evaluation of Cell Structure (3)>

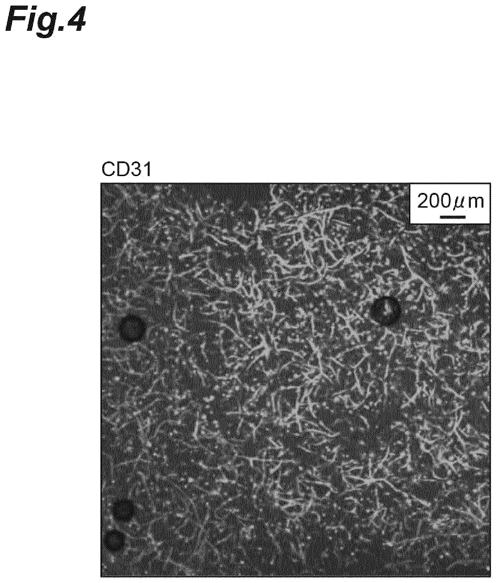

An attempt was made to produce a cell structure with the same method as in Test Example 2, except that no mature adipocyte was used, and 2 mg of sCMF, 0.6 mg of fibrinogen, and 0.3 U of thrombin were used, and as a result a cell structure in which a vascular network was formed was successfully produced. Figure 4 shows the result of fluorescence imaging (4× magnification) for the cell structure in which only the blood vessels were stained with an anti-CD31 antibody. It was demonstrated that a cell structure with a vascular network can be produced even if no mature adipocyte is used.

<Test Example 5: Production and Evaluation of Cell Structure (4)>

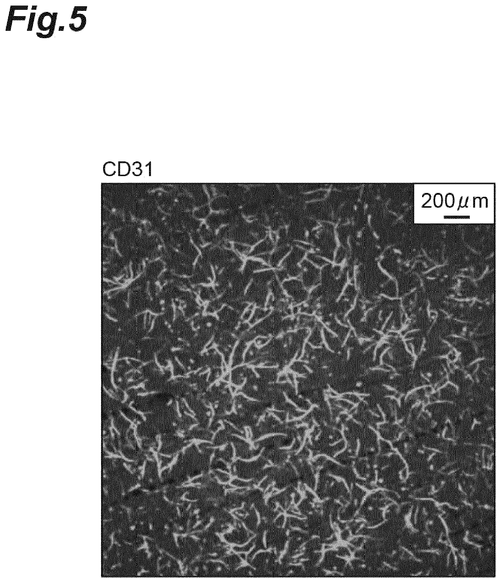

An attempt was made to produce a cell structure with the same method as in Test Example 2, except that no mature adipocyte was used, and 50000 cells of HUVECs (ADSCs:HUVECs = 5:1), 2 mg of sCMF, 0.6 mg of fibrinogen, and 0.3 U of thrombin were used, and as a result a cell structure in which a vascular network was formed was successfully produced. Figure 5 shows the result of fluorescence imaging (4× magnification) for the cell structure in which only the blood vessels were stained with an anti-CD31 antibody. It was demonstrated that a cell structure with a vascular network can be produced even if no mature adipocyte is used and the ratio of ADSCs and HUVECs is changed.

<Test Example 6: Production and Evaluation of Cell Structure (5)>

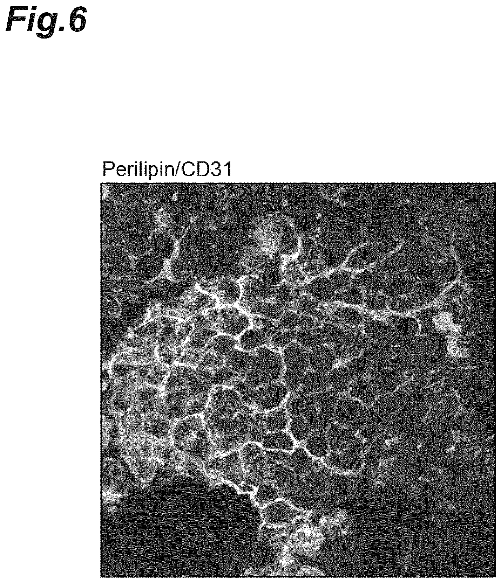

A cell structure was produced with the same method as in Test Example 2, except that adipose tissue obtained from the femur of a human (biological tissue) through liposuction was used in place of mature adipocytes, ADSCs, and HUVECs, and the total volume before seeding was adjusted to 60 μL. The adipose tissue was made into a liquid form by finely cutting 3 g of the collected biological tissue into pieces of about 1 to 3 mm in size by using scissors and tweezers and slowly pipetting them several times with a 10-mL syringe. A 60-μL portion was taken from the adipose tissue made into a liquid form, and mixed with sCMF. It should be noted that adipose tissue obtained through liposuction contains mature adipocytes, adipose stem cells, and vascular endothelial cells. Figure 6 shows the result of fluorescence imaging (10× magnification) for the cell structure stained with the same method as in Test Example 2. It was demonstrated that a cell structure with a vascular network can be produced even if adipose tissue obtained from biological tissue is used in place of mature adipocytes, ADSCs, and HUVECs. It was confirmed for a case with use of 2 mg of sCMF that a cell structure with a vascular network can be produced similarly.

While it was confirmed in all of Test Examples 2 to 6 that a cell structure with a vascular network can be produced, comparison among Test Examples 2 to 6 found that a cell structure with a vascular network more similar to that of biological tissue was successfully produced as a tendency when 1 mg of sCMF was used than when 2 mg of sCMF was used. In addition, a cell structure with a vascular network more similar to that of biological tissue was successfully produced as a tendency when the ratio of ADSCs and HUVECs was ADSCs:HUVECs = 2:1 than when it was ADSCs:HUVECs = 5:1.

<Comparative Example>

A cell structure was produced with the same method as in Test Example 2, except that sCMF was not used, and 1 mg of fibrinogen and 0.5 U of thrombin were used. No vascular formation was observed in the cell structure produced. Further, a cell structure was produced with the same method as in Test Example 2, except that no ADSC was included. In the cell structure produced, only slight vascular formation was found, and formation of a vascular network surrounding mature adipocytes was not found.

<Test Example 7: Production and Evaluation of Cell Structure (6)>

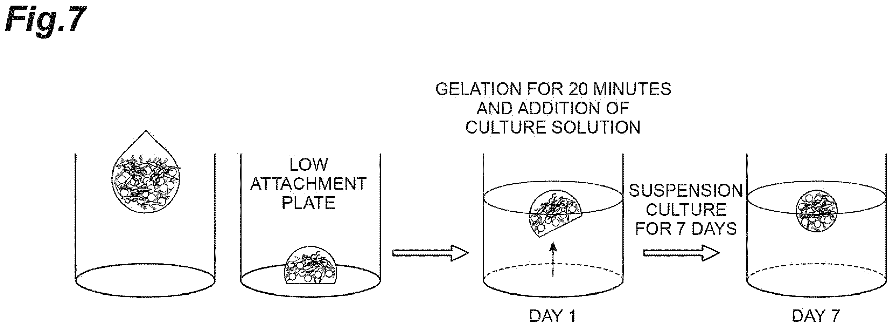

Cells, reagents, culture media, and solutions for use in production of a cell structure were prepared as in Test Example 2. A defibered collagen component (sCMF) produced with the same method as in Test Example 1 was used. The summary of the present test example is shown in Figure 7. In the droplet in the left in Figure 7, circles represent mature adipocytes, open rhombuses represent ADSCs, and gray short rods represent HUVECs.

(Production Method)

A fragment of human adipose tissue was washed with PBS containing 5% antibiotic. Into six wells in a 6-well plate, 4 to 6 g of the tissue was distributed. In 2 mL of 2 mg/mL collagenase solution, the tissues were finely cut into pieces of about 1 to 3 mm in size by using scissors and tweezers. After incubation at 37°C and 230 rpm for 1 hour, mixing was performed with a 10-mL pipette for 30 minutes. Each lysate was filtered through an iron mesh of 500 μm in pore size, and 2 mL of DMEM was added to each well to recover all the cells digested, which was then centrifuged at 200 g under room temperature (15 to 25°C) for 3 minutes. Mature adipocytes are contained in the upper layer, a yellow oily layer, and adipose stem cells and hemocytes are contained in the pellet. The medium between the upper layer and the lower layer was sucked and discarded by using a long needle and a 10-mL syringe, and the mature adipocytes contained in the upper layer and the adipose stem cells and hemocytes contained in the lower layer were each washed twice with 25 mL of PBS (5% BSA, 1% antibiotic). In the washing, centrifugation was performed in the same manner as described above to separate into three layers of an upper layer, a lower layer, and a medium between the upper layer and the lower layer, and the medium between the upper layer and the lower layer was sucked and discarded. After washing was performed twice with 25 mL of PBS (5% BSA, 1% antibiotic), washing was performed with 25 mL of DMEM.

Only the upper layer containing mature adipocytes was collected, and aliquoted in Eppendorf tubes. The nuclei were stained by Hoechst staining (1000-fold diluted Hoechst, staining for 10 minutes), and the cell count was determined on a Turker Burk hemocytometer through a fluorescence microscope.

The pellet containing ADSCs was suspended in 10 mL of DMEM, seeded in a 10-cm dish, and subcultured. The ADSCs were separated from the dish by using trypsin/EDTA and suspended in 1 mL of DMEM, and the cell count was determined.

HUVECs purchased from Lonza were suspended in 10 mL of DMEM, seeded in a 10-cm dish, and subcultured. The HUVECs were separated from the dish by using trypsin/EDTA and suspended in 1 mL of DMEM, and the cell count was determined.

In order to finally obtain one generally spherical cell structure (hereinafter, also referred to as a "cell ball") with a diameter of approximately 1 mm, 16250 cells of mature adipocytes, 13750 cells of ADSCs, 6875 cells of HUVECs, 0.06 mg of sCMF, 0.04 mg of fibrinogen, and 0.02 U of thrombin were used.

Weighed was 2.4 mg of sCMF, to which 1 mL of DMEM was added, and the resultant was gently mixed until a situation that only small particles of sCMF were observed was achieved. Centrifugation was performed at 10000 rpm under room temperature for 1 minute, and the supernatant was sucked to afford an sCMF pellet. On the sCMF pellet, 220000 cells of ADSCs and 275000 cells of HUVECs were gently added, centrifugation was performed at 3500 rpm under room temperature for 1 minute, and the supernatant was sucked. Fibrinogen (32 $\mu$L of 50 mg/mL fibrinogen stock solution) was added, and gently mixed with the cells and sCMF. Further, 650000 cells of mature adipocytes were added and gently mixed. A small volume of DMEM was added to adjust the total volume to 200 $\mu$L. Immediately, thrombin (3.9 $\mu$L of 202 U/mL thrombin stock solution) was added and slightly mixed, and then 5 $\mu$L (equivalent to one cell ball) of the mixture (equivalent to 40 cell balls) was seeded on each well of a low attachment 96-well plate (IWAKI #4860-800LP).

Incubation was performed in an incubator at 37°C for 15 minutes to cause gelation, and 300 $\mu$L of EGM-2 culture medium containing insulin at a final concentration of 10 $\mu$g/mL was added.

Culturing for 24 hours was followed by transfer to a low attachment 24-well plate (IWAKI #4820-800LP), and 2 mL of the above EGM-2 culture medium was added to pull apart from the plate. The culture medium was replaced every 2 to 3 days until on day 7 after the beginning of culturing.

Fluorescence imaging for the cell structure was carried out with the following procedure. The Transwell containing the cell structure obtained was transferred to a 24-well plate (IWAKI #4820-800LP). After washing with 200 $\mu$L of PBS, the tissue was fixed by using 200 $\mu$L of 4% PFA under 4°C overnight. Washing was performed three times with 200 $\mu$L of PBS. For immunostaining, the cells were permeabilized with 200 $\mu$L of 0.05% Triton/PBS under room temperature for 7 minutes, and washed three times with 200 $\mu$L of PBS.

Blocking was performed with 200 $\mu$L of 1% BSA/PBS solution under room temperature for 1 hour. The BSA solution was sucked, and 100 $\mu$L of primary antibody solution (CD31 and perilipin 100-fold diluted with 1% BSA/PBS solution) was added. A wet wipe was laid beneath the plate, and the plate was covered with an aluminum foil and incubated under 4°C overnight. After washing was performed three times with 200 $\mu$L of PBS, 100 $\mu$L of secondary antibody solution (AlexaFluor647-labeled anti-mouse anti-CD31 antibody and AlexaFluor488-labeled anti-rabbit anti-perilipin antibody 200-fold diluted with 1% BSA/PBS solution, 1000-fold diluted Hoechst) was added. A wet wipe was laid beneath the plate, and the plate was covered with an aluminum foil and incubated under room temperature for 2 hours, and then washed four times with 200 $\mu$L of PBS.

The cell balls were directly disposed on a complete plate of the confocal quantitative image cytometer CQ1 (manufactured by Yokogawa Electric Corporation), and the stained cell structures were observed by using the confocal quantitative image cytometer CQ1 with laser excitation lights at 640 nm (AlexaFluor647) and 488 nm (AlexaFluor488).

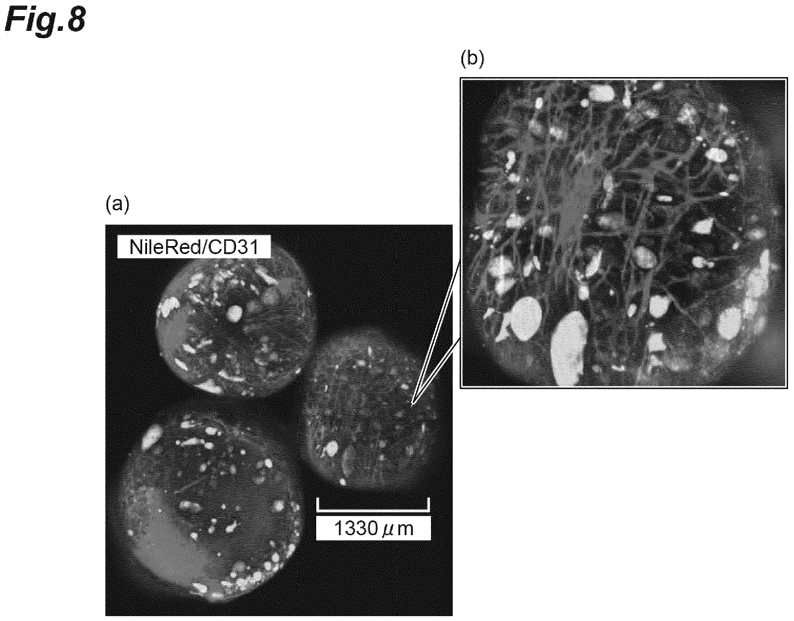

Figure 8 shows the result of fluorescence imaging for the cell balls with a vascular network with Nile Red staining and CD31 staining. CD31 staining was carried out in the same manner as in Test Example 2, and Nile Red staining was carried out by using a conventional method. The photograph in (b) shows a further enlarged view of one of the cell balls in (a). It was confirmed that, like the biological tissue, a vascular network was formed in such a way to surround mature adipocytes (large, round, eye-shaped lipid droplets) in the cell balls. Such a vascular network was formed even in the inside of each cell ball.

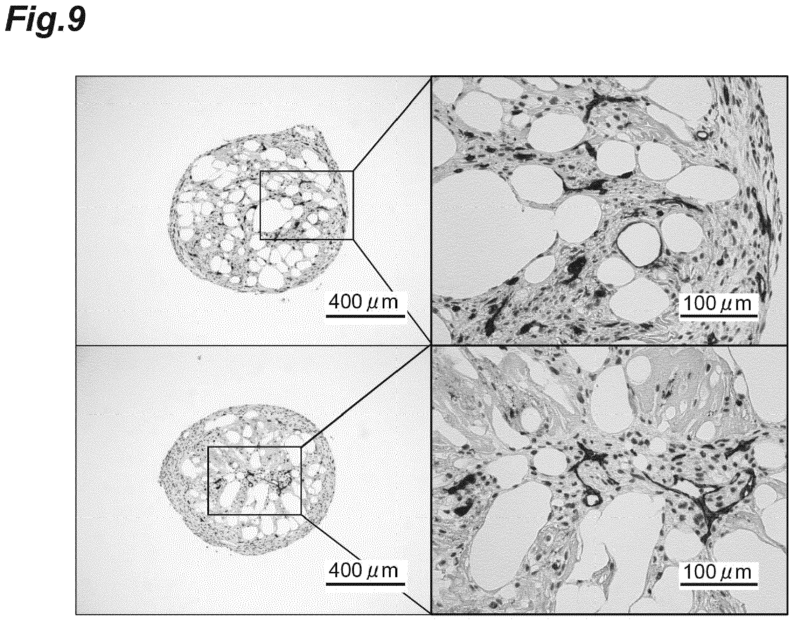

Figure 9 shows the result of observation for sections of the cell balls with a vascular network with CD31 immunohistological staining. Also, from this result, in which many lumens were observed in the cell balls, it was confirmed that a vascular network was formed even in the inside of each cell structure.

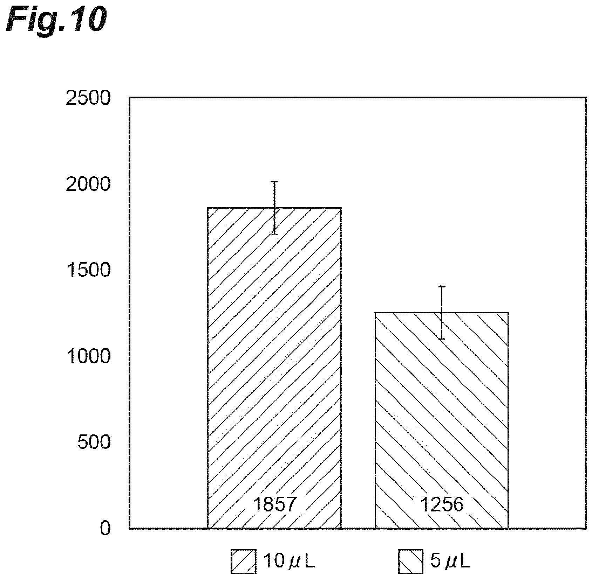

Figure 10 shows average diameters (n = 12 cell balls/volume) of the cell balls produced by using the method of the present test example after culturing for 7 days. The average diameter of the cell balls of 5 $\mu$L was 1256 $\mu$m, and the average diameter of the cell balls of 10 $\mu$L was 1857 $\mu$m.

<Test Example 8: Production and Evaluation of Cellular Tissue Including Plurality of Cell Balls>

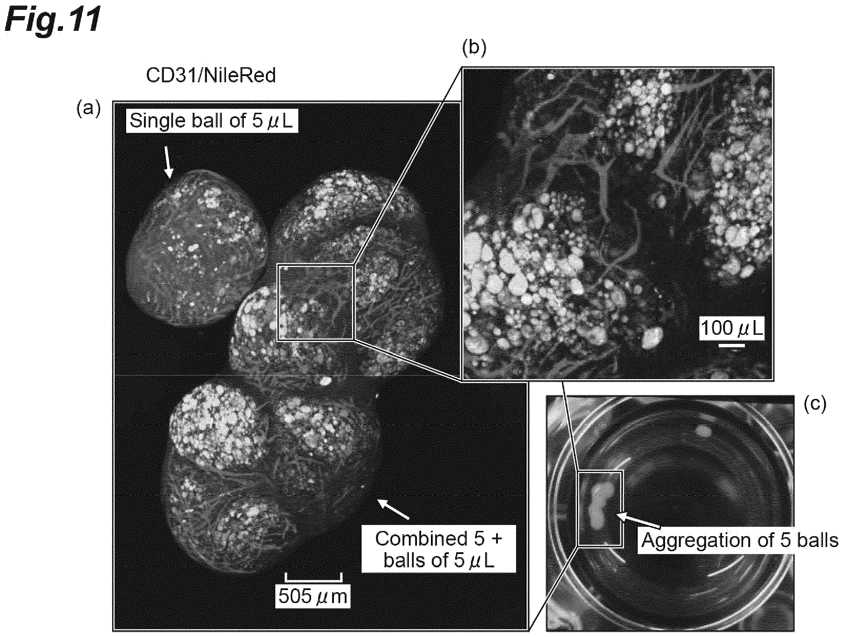

A plurality of the cell balls of 5 $\mu$L 7 days after the beginning of culturing, produced in Test Example 7, was neighbored to contact with each other, and suspension-cultured in the state in 10 mL of culture solution for 7 days. Observation was performed with staining in the same manner as in Test Example 7. The result showed that, as demonstrated in Figure 11, cellular tissue in which a plurality of cell balls (five cell balls in Figure 11) aggregated to combine together was obtained. It was confirmed for the cellular tissue, in which five cell balls combined together, not only that vascular networks were connected together in each cell ball, but also that vascular networks were connected together among a plurality of cell balls (Figure 11(a) and (b)).

<Test Example 9: Transplantation Test and Evaluation for Cell Balls>

Six individuals of immunodeficient mice were prepared, the back skin of each was incised, and any of the following (1) to (3) was flowed into the incised part, which was sutured and then three individuals were grown for 30 days and three individuals were grown for 90 days.

(1) A mixture obtained by suspending 111 cell balls after culturing, produced in Test Example 7, in 100 $\mu$L of a solution containing 2.5 mg of fibrinogen and 1.25 U of thrombin

(2) A mixture obtained by suspending 111 cell balls produced in the same manner as for (1), except that no HUVEC was used, in 100 $\mu$L of a solution containing 2.5 mg of fibrinogen and 1.25 U of thrombin

(3) A cell structure (100 $\mu$L) produced by using the adipose tissue obtained from the femur of a human (biological tissue) through liposuction in Test Example 6

Tissue was collected from the transplant part of each individual after growing for 30 days. Adipose tissue was formed at the part with transplantation of the above (2), whereas formation of a vascular network was not found. Adipose tissue was formed at the part with transplantation of the above (3) and a vascular network was slightly found in the tissue surface, but the presence of a large oil droplet was also found. Formation of an oil droplet indicates death of an adipocyte. At the part with transplantation of the above (1), which used cell balls produced in Test Example 7, on the other hand, formation of adipose tissue with a vascular network spreading in the tissue surface was found. In addition, formation of an oil droplet was not found in the tissue at the part with transplantation of the above (1), and thus much superiority in post-transplantation fixability was demonstrated.

Tissue was collected from the part with transplantation of the above (1) in the individual after growing for 30 days, and subjected to perilipin staining and DAPI staining as Test Example 2 (Figure 12). DAPI staining was carried out by using a conventional method. A:SFT shows tissue collected from the part with transplantation of the above (3), and C:3DVFT shows tissue collected from the part with transplantation of the above (1). In Figure 12, the top images show results of bright-field observation, the middle images show results with perilipin staining, and the bottom images show results with DAPI staining. It was confirmed that mature adipocytes were formed in the tissue formed at the transplant part.

Tissue was collected from the part with transplantation of the above (1) in the individual after growing for 90 days, and subjected to perilipin staining and CD31 staining as Test Example 2 (Figure 13). In Figure 13, the top images show results of bright-field observation, the middle images show results with CD31 staining, and the bottom images show results with DAPI staining. It was confirmed that not only mature adipocytes were formed in the tissue formed at the transplant part, but also a vascular network was formed.

From the above, the cell structure according to the present embodiment was demonstrated to have superior post-transplantation fixability, thus being suitable for transplantation.

<Test Example 10: Production of Cell Structure and Evaluation of Glucose Uptake>

(Production of Cell structure)

A cell structure provided with a vascular network was produced with the same procedure as in Test Example 2, except that 6500 cells of mature adipocytes, 5500 cells of adipose stem cells, 2750 cells of vascular endothelial cells, 0.025 mg of the defibered collagen component (sCMF), 0.015 mg of fibrinogen, and 0.007 U of thrombin were used per tissue.

The materials were prepared for 96 wells all at once to afford a gel, which was seeded on each well of a 96-well plate. Two types of sCMF, sCMF produced only with a sponge fragment of porcine skin-derived collagen I (manufactured by NH Foods Ltd.) and sCMF produced with a mixture of a sponge fragment of porcine skin-derived collagen I and a sponge fragment of porcine skin-derived collagen III (manufactured by NH Foods Ltd.), were used. The mixing ratio of collagen I and collagen III in the mixture is inferred to be about 8:2. In the present test example, sCMF produced with a mixture of a sponge fragment of porcine skin-derived collagen I and a sponge fragment of porcine skin-derived collagen III was used.

The cell structures after seeding were cultured until a day to conduct uptake-release test. DMEM culture medium containing neither glucose nor fatty acid was used as the culture medium, and replacement of the culture medium was carried out once per 2 days.

(Evaluation of Glucose Uptake)

Test to evaluate uptake of glucose was conducted on day 7 and day 14 after the beginning of forming cell structures.

Before the test, the culture medium was replaced with 300 $\mu$L of DMEM culture medium containing no glucose. The cell structures were incubated for 6 hours, thereby starving the cell structures. The culture medium was replaced with 100 $\mu$L of DMEM culture medium containing 0.1 mg/mL NBD-modified glucose (2-deoxy-2-[(7-nitro-2,1,3-benzoxadiazol-4-yl)amino]-D-glucose, Cayman Chemical Company). The cell structures were incubated, and fluorescence measurement was carried out after 10 minutes, 30 minutes, 60 minutes, 120 minutes, and 150 minutes. Before the measurement at each timing, the culture medium for a cell structure to be subjected to measurement was substituted with 300 μL of PBS buffer, and the fluorescence was measured with a plate reader (Synergy HTX, manufactured by BioTek Instruments, Inc.) to determine the cumulative value of fluorescence intensity in the image.

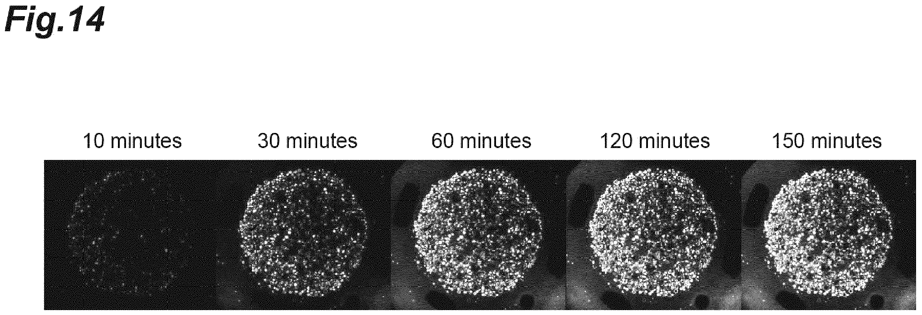

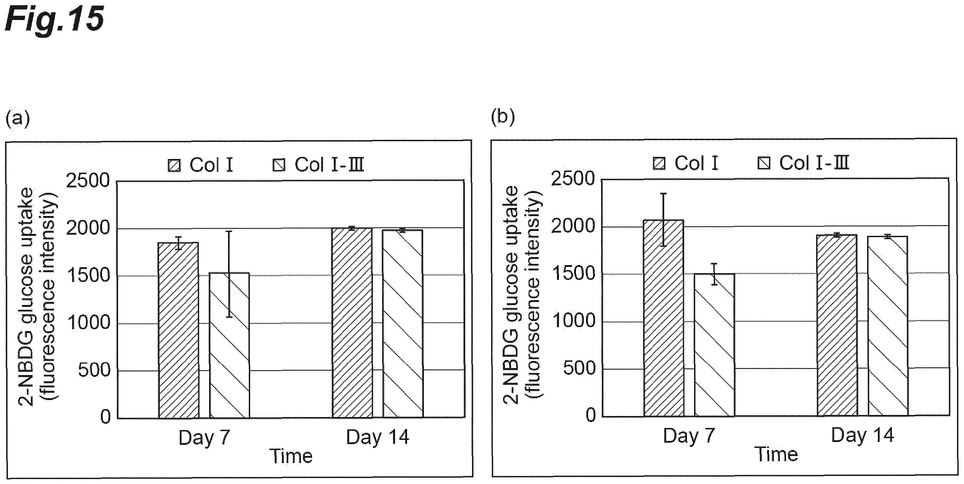

As demonstrated in Figure 14, it was confirmed that the longer the time to expose a cell structure to glucose is, the more glucose is taken in the construct. Comparison of fluorescence intensity after 60 minutes on day 7 and day 14 after the beginning of formation is shown in the graph in (b) of Figure 15. The number of samples for each treatment was 4 (n = 4), and glucose uptake after 0 hours of treatment in a control culture medium containing no NBD-modified glucose was defined as 100%. From these results, it was found that the glucose uptake ability of the cell structure is retained for 14 days or more. The graph in (a) of Figure 15 shows results with a tissue product produced in the same manner as the cell structure in the present test example, except that no vascular endothelial cell was included, and, similarly, it was found that the glucose uptake ability is retained for 14 days or more.

<Test Example 11: Evaluation of Effects of Uptake Inhibitor and Promoter for Glucose>

A tissue product including no vascular endothelial cell was produced in the same manner as in Test Example 10, and evaluation was carried out with the tissue product on day 7 after the beginning of formation thereof. sCMF produced by using a mixture of a sponge fragment of porcine skin-derived collagen I and a sponge fragment of porcine skin-derived collagen III was used.

Into the DMEM culture medium containing NBD-modified glucose in (Evaluation of Glucose Uptake) of Test Example 10, 125 μM apigenin as an uptake inhibitor for glucose or 10 μg/mL insulin as an uptake promoter for glucose was introduced. The tissue product was incubated, and fluorescence measurement was carried out after 20 minutes, 45 minutes, 70 minutes, 90 minutes, and 135 minutes in the same manner as in Test Example 10.

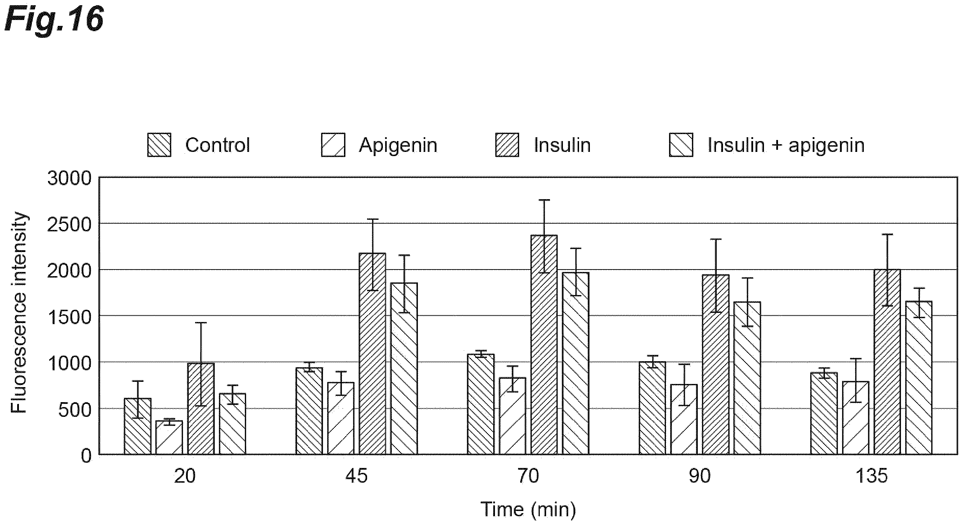

The results are shown in Figure 16. The number of samples for each treatment was 5 (n = 5), and glucose uptake in the same control culture medium as in Test Example 10, which contained neither apigenin nor insulin, was defined as 100%. It was found that the amount of uptake of glucose was reduced by addition of apigenin, and the amount of uptake of glucose increased by addition of insulin. When both apigenin and insulin were added, the amount of glucose uptake increased, whereas the increase level was smaller than that when insulin alone was added, and hence it was suggested that the increase level of the amount of glucose uptake is controlled by the concentrations of the drugs.

<Test Example 12: Evaluation of Effect of Uptake Promoter for Glucose and Fatty Acid>

A tissue product including no vascular endothelial cell was produced in the same manner as in Test Example 10, and evaluation was carried out with the tissue product on day 7 after the beginning of formation thereof. sCMF produced by using a mixture of a sponge fragment of porcine skin-derived collagen I and a sponge fragment of porcine skin-derived collagen III was used.

A tissue product was incubated in the same manner as in Test Example 10, except that, in place of NBD-modified glucose in Test Example 10, 4 μM BODIPY-modified fatty acid (Invitrogen (R) BODIPY (R) 500/510 C1, C12 (4,4-Difluoro-5-Methyl-4-Bora-3a,4a-Diaza-s-Indacene-3-Dodecanoic Acid), Thermo Fisher Scientific) was added to DMEM culture medium, and fluorescence measurement was carried out after 5 minute, 30 minutes, and 60 minutes. Further, 10 μg/mL insulin was added to the DMEM culture medium containing 4 μM BODIPY-modified fatty acid, and fluorescence measurement was carried out in the same manner.

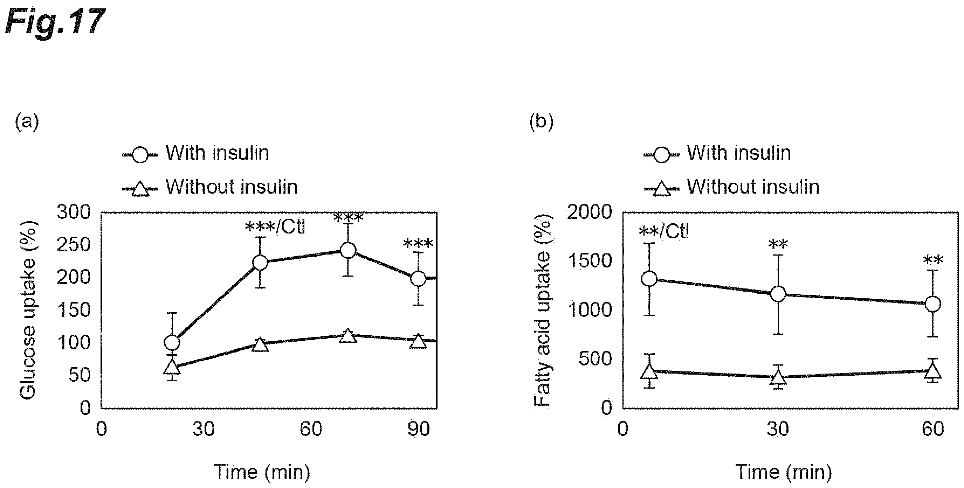

The results are shown in Figure 17. The graph in (b) of Figure 17 shows the variation of the amount of fatty acid uptake in Test Example 12 herein. The number of samples for each treatment was 5 (n = 5), and fatty acid uptake in the same control culture medium as in Test Example 10, which contained no insulin, was defined as 100%. From these results, it was demonstrated that uptake of not only glucose but also fatty acid is promoted by insulin.

The graph in (a) of Figure 17 shows the variation of the amount of glucose uptake in Test Example 11. Fluorescence measurement was carried out 20 minutes, 45 minutes, 70 minutes, and 90 minutes after the beginning of incubation of the tissue product. The number of samples for each treatment was 5 (n = 5), and glucose uptake in the same control culture medium as in Test Example 10, which contained no insulin, was defined as 100%. Comparison between the graphs in (a) and (b) suggested the possibility that the amount of uptake of fatty acid saturates more quickly than that of glucose.

<Test Example 13: Evaluation of Effect of Uptake Inhibitor for Fatty Acid>

A cell structure including vascular endothelial cells and a tissue product including no vascular endothelial cell were produced in the same manner as in Test Example 10, and evaluation was carried out with the cell structure and tissue product on day 14 after the beginning of formation thereof. sCMF produced by using a sponge fragment of porcine skin-derived collagen I was used.

Into DMEM culture medium containing 4 $\mu$M BODIPY-modified fatty acid in place of NBD-modified glucose in (Evaluation of Glucose Uptake) of Test Example 10, 100 ng/mL TNF$\alpha$, as an uptake inhibitor for fatty acid was introduced. The cell structure including vascular endothelial cells and the tissue product including no vascular endothelial cell were each incubated, and fluorescence measurement was carried out after 0 minutes, 5 minutes, 30 minutes, and 60 minutes in the same manner as in Test Example 10. The number of samples for each treatment was 5 (n = 5), and fatty acid uptake in the same control culture medium as in Test Example 10, which contained no TNF$\alpha$, was defined as 100%.

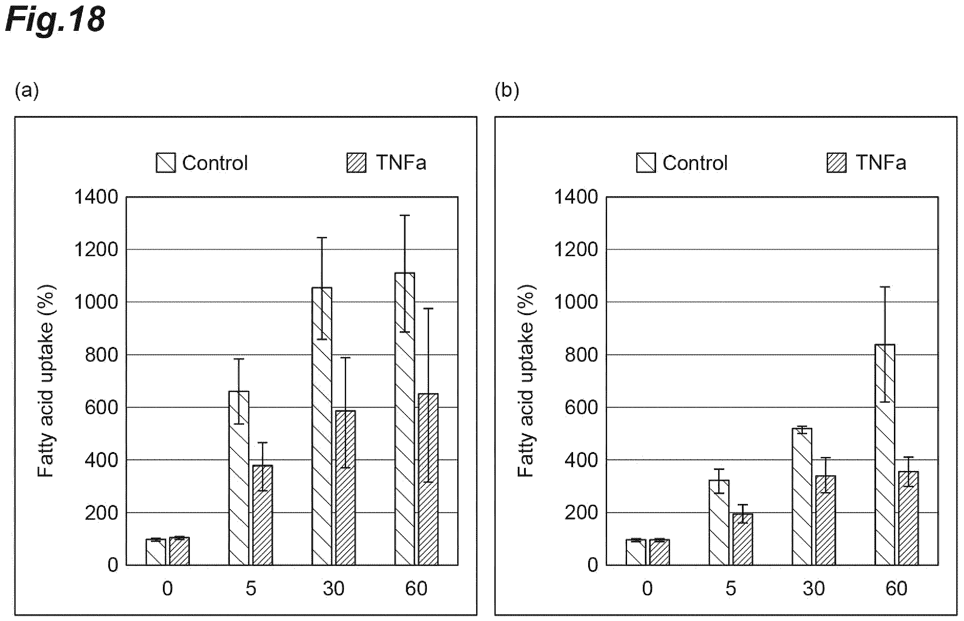

The results are shown in Figure 18. In Figure 18, the graph in (b) shows the result with the cell structure including vascular endothelial cells, and the graph in (a) shows the result with the tissue product including no vascular endothelial cell. It was found that the amount of uptake of fatty acid is reduced by addition of TNF$\alpha$.

<Test Example 14: Evaluation of Glucose and Fatty Acid Release and Evaluation of Effect of Release Promoter for Fatty Acid>

A tissue product was produced in the same manner as for the cell structure in Test Example 10, except that no vascular endothelial cell was included, and evaluation was carried out with the tissue product on day 14 after the beginning of formation thereof.

In order to measure the amount of release of glucose once taken up, incubation was performed on DMEM culture medium supplemented with 0.1 mg/mL NBD-modified glucose as in Test Example 10 for 60 minutes, and then the culture medium was substituted again with 300 $\mu$L of DMEM culture medium supplemented with NBD-modified glucose, or with 300 $\mu$L of DMEM culture medium supplemented with 2 mM isoproterenol and NBD-modified glucose. Thereafter, incubation was performed for 30 minutes to allow glucose to be released from the cellular tissue, and fluorescence measurement was carried out.

Similarly, in order to measure the amount of release of fatty acid once taken up, incubation was performed on DMEM culture medium supplemented with 4 $\mu$M BODIPY-modified fatty acid as in Test Example 12 for 60 minutes, and then the culture medium was substituted again with 300 $\mu$L of DMEM culture medium supplemented with BODIPY-modified fatty acid, or with 300 $\mu$L of DMEM culture medium supplemented with 2 mM isoproterenol and BODIPY-modified fatty acid. Thereafter, incubation was performed for 30 minutes to allow fatty acid to be released from the cellular tissue, and fluorescence measurement was carried out.

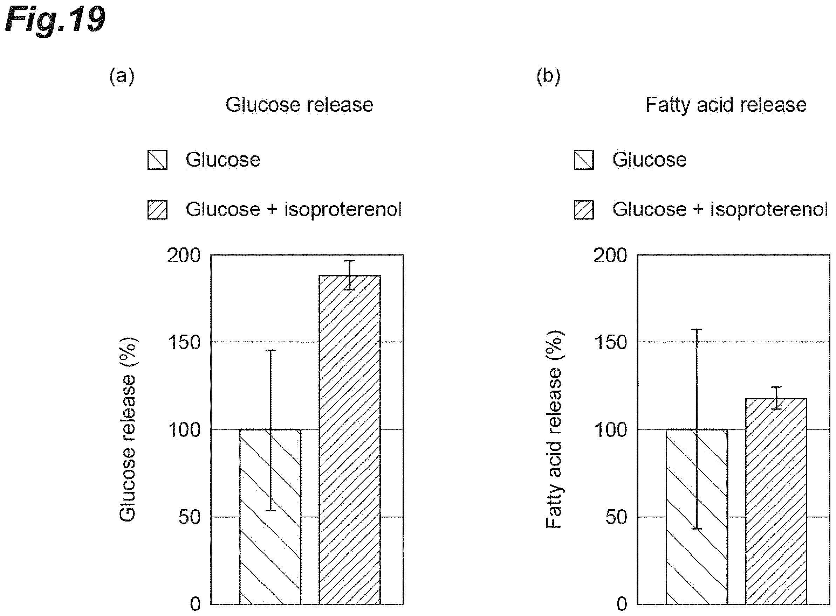

The results are shown in Figure 19. The graph in (a) shows comparison of the amount of release of glucose, and the graph in (b) shows comparison of the amount of release of fatty acid. The number of samples for each treatment was 5 (n = 3), fluorescence intensity for the tissue product with the culture medium containing no isoproterenol was defined as 100%, and what % of the amount of uptake was released was determined by comparing with fluorescence intensity for the tissue product with the culture medium containing isoproterenol. The amount of uptake for the culture medium containing no isoproterenol is expected not to change very much through incubation for 30 minutes.

It was found that, for the case with replacement with the culture medium containing isoproterenol, the amount of glucose or fatty acid that had been taken up was reduced after incubation for 30 minutes. That is, it was demonstrated that release is promoted by addition of isoproterenol for both glucose and fatty acid.

## Claims

1. A cell structure comprising: a fragmented extracellular matrix component; and cells, wherein

    the cell structure comprises an intercellular vascular network, and
    the cells comprise at least adipocytes and vascular endothelial cells.

2. The cell structure according to claim 1, wherein the vascular network is formed among the adipocytes.

3. The cell structure according to claim 1 or 2, wherein the adipocytes comprise mature adipocytes.

4. The cell structure according to any one of claims 1 to 3, wherein an average length of the fragmented extracellular matrix component is 100 nm or more and 400 $\mu$m or less.

5. The cell structure according to any one of claims 1 to 4, wherein a content of the extracellular matrix component in the cell structure is 0.01 to 90% by mass based on a dry mass of the cell structure.

6. The cell structure according to any one of claims 1 to 5, wherein the fragmented extracellular matrix component comprises collagen.

7. The cell structure according to any one of claims 1 to 6, further comprising fibrin.

8. The cell structure according to any one of claims 1 to 6, for transplantation.

9. A method for producing a cell structure comprising an intercellular vascular network, the method comprising:

a contacting step of bringing a fragmented extracellular matrix component and cells into contact with each other, wherein the cells (i) comprise at least adipocytes, stem cells, and vascular endothelial cells, or (ii) comprise at least adipose stem cells and vascular endothelial cells; and
a culturing step of culturing the cells that are in contact with a fragmented extracellular matrix.

10. The method according to claim 9, wherein the cells comprise adipocytes, adipose stem cells, and vascular endothelial cells.

11. The method according to claim 9 or 10, wherein the adipocytes comprise mature adipocytes.

12. The method according to any one of claims 9 to 11, wherein an amount of the fragmented extracellular matrix component in the contacting step is 0.1 to 100 mg per $1.0 \times 10^6$ cells.

13. The method according to any one of claims 9 to 12, wherein a cell count ratio between the stem cells and the vascular endothelial cells in the contacting step is 100/1 to 1/100.

14. The method according to any one of claims 9 to 13, wherein the fragmented extracellular matrix component comprises collagen.

15. The method according to any one of claims 9 to 14, the method further comprising adding fibrinogen in the contacting step, or after the contacting step before the culturing step.

16. A non-human model animal comprising the cell structure according to any one of claims 1 to 8 as an implant.

17. A method for producing a non-human model animal, comprising transplanting the cell structure according to any one of claims 1 to 8 into a non-human animal.

18. A method for transplanting a cell structure having vascular structure, the method comprising transplanting the cell structure according to any one of claims 1 to 8 into an animal.

19. A cell structure comprising: a fragmented extracellular matrix component; and cells, wherein

the cell structure comprises an intercellular vascular network,
the cell structure is a cell aggregate formed without adhering to a support, and
the cells comprise at least adipocytes and vascular endothelial cells.

20. The cell structure according to claim 19, being generally spherical.

21. A method for producing a cell structure comprising an intercellular vascular network, the method comprising:

a contacting step of bringing a fragmented extracellular matrix component and cells into contact with each other, wherein the cells (i) comprise at least adipocytes, stem cells, and vascular endothelial cells, or (ii) comprise at least adipose stem cells and vascular endothelial cells; and
a culturing step of culturing the cells that are in contact with a fragmented extracellular matrix, wherein the culturing step comprises culturing the cells that are in contact with the fragmented extracellular matrix without the cells adhering to a support.

22. The method according to claim 21, wherein the culturing step comprises separating the cells that are in contact with the fragmented extracellular matrix apart from the support.

23. A cellular tissue comprising a plurality of the cell structures according to claim 19 or 20, wherein the vascular networks are connected among the plurality of the cell structures.

24. A method for producing a cellular tissue, comprising suspension-culturing a plurality of the cell structures according to claim 19 or 20.

25. A method for producing a non-human model animal, comprising transplanting a plurality of the cell structures according to claim 19 or 20 into a non-human animal.

26. The method according to claim 25, comprising growing the cell structures for 30 days or more after transplanting into the non-human animal.

27. The method according to claim 26, comprising growing the cell structures for 90 days or more after transplanting into the non-human animal.

28. A method for evaluating an effect of a drug for inhibiting or promoting metabolism in adipose tissue, by using the cell structure according to any one of claims 1 to 8, 19, and 20.

29. The method according to claim 28, the method comprising:

an administration step of administering the drug for inhibiting or promoting metabolism in adipose tissue to the cell structure; and
an evaluation step of evaluating the effect of the drug based on change in metabolism in the cell structure receiving administration of the drug.

30. The method according to claim 29, wherein the evaluation step comprises
evaluating the effect of the drug using, as an index, change in uptake of glucose and/or fatty acid and/or change in release of glucose and/or fatty acid taken up.

31. A method for screening for a drug for inhibiting or promoting metabolism in adipose tissue, by using the cell structure according to any one of claims 1 to 8, 19, and 20.

32. The method according to claim 31, the method comprising:

a step of measuring metabolism in the cell structure receiving administration of the drug; and
a step of comparing metabolism in the cell structure receiving administration of the drug with metabolism in the cell structure not receiving administration of the drug, and, if the metabolism in the cell structure receiving administration of the drug is lower, selecting the drug as a candidate substance for a drug for inhibiting metabolism in adipose tissue, or comprising:

a step of measuring metabolism in the cell structure receiving administration of the drug; and
a step of comparing metabolism in the cell structure receiving administration of the drug with metabolism in the cell structure not receiving administration of the drug, and, if the metabolism in the cell structure receiving administration of the drug is higher, selecting the drug as a candidate substance for a drug to promote metabolism in adipose tissue.

33. The method according to claim 32, wherein the comparison of metabolism in the cell structure is carried out using, as an index, uptake of glucose and/or fatty acid and/or release of glucose and/or fatty acid taken up.

## *Fig.1*

(a)

(b)

Perilipin/CD31

100μm

Fig.2

*Fig.3*

Perilipin/CD31

*Fig.4*

CD31

200μm

**Fig.5**

CD31

# Fig.6

Perilipin/CD31

# Fig.7

EP 3 950 711 A1

**Fig.8**

(b)

(a)

NileRed/CD31

1330 μm

EP 3 950 711 A1

Fig.9

# Fig.10

*Fig.11*

CD31/NileRed

(a)

Single ball of 5 μL

(b)

100 μL

Combined 5 + balls of 5 μL

505 μm

(c)

Aggregation of 5 balls

EP 3 950 711 A1

*Fig.12*

A:SFT          C:3DVFT

## Fig.13

A:SFT          C:3DVFT

Fig.14

**Fig.15**

(a)

(b)

Fig.16

Fig.17

**Fig.18**

(a)

(b)

EP 3 950 711 A1

## Fig.19

(a)

Glucose release

⬜ Glucose

▨ Glucose + isoproterenol

(b)

Fatty acid release

⬜ Glucose

▨ Glucose + isoproterenol

EP 3 950 711 A1

## INTERNATIONAL SEARCH REPORT

International application No.

PCT/JP2020/012476

### A. CLASSIFICATION OF SUBJECT MATTER

C07K 14/78(2006.01)i; C12N 5/071(2010.01)i; C12N 5/074(2010.01)i; C12N 5/077(2010.01)i; C12Q 1/02(2006.01)i; G01N 33/15(2006.01)i
FI: C12N5/071; C07K14/78; C12N5/074; C12N5/077; G01N33/15 Z; C12Q1/02

According to International Patent Classification (IPC) or to both national classification and IPC

### B. FIELDS SEARCHED

Minimum documentation searched (classification system followed by classification symbols)
C07K14/78; C12N5/071; C12N5/074; C12N5/077; C12Q1/02; G01N33/15

Documentation searched other than minimum documentation to the extent that such documents are included in the fields searched
Published examined utility model applications of Japan      1922-1996
Published unexamined utility model applications of Japan    1971-2020
Registered utility model specifications of Japan            1996-2020
Published registered utility model applications of Japan    1994-2020

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

### C. DOCUMENTS CONSIDERED TO BE RELEVANT

| Category* | Citation of document, with indication, where appropriate, of the relevant passages | Relevant to claim No. |
|---|---|---|
| X | CHOI, J. S. et al., "Human extracellular matrix (ECM) powders for injectable cell delivery and adipose tissue engineering", Journal of Controlled Release, 2009, vol. 139, pp. 2-7, in particular, abstract, fig. 1, 4 | 1-3,5-8,16-20,25-27 |
| Y | in particular, abstract, fig. 1, 4 | 4,28-33 |
| Y | LOUIS, F. et al., "3D collagen microfibers stimulate the functionality of preadipocytes and maintain the phenotype of mature adipocytes for long term cultures", Acta Biomaterialia, 28 November 2018, vol. 84, pp. 194-207, in particular, abstract, page 198, right column, paragraph [0001], fig. 5, 6 | 4,28-33 |

☒ Further documents are listed in the continuation of Box C.    ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 12 May 2020 (12.05.2020) | 26 May 2020 (26.05.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| International application No. |
| --- |
| PCT/JP2020/012476 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | WO 2018/143286 A1 (TOPPAN PRINTING CO., LTD.) 09.08.2018 (2018-08-09) in particular, claims 1-15, examples, fig. 5 | 1-33 |
| Y | XIE, Q. et al., "Hypoxia enhances angiogenesis in an adipose-derived stromal cell/endothelial cell co-culture 3D gel model", Cell Proliferation, 2016, vol. 49, pp. 236-245, in particular, abstract, fig. 1 | 1-33 |
| Y | WITTMANN, K. et al., "Engineering Vascularized Adipose Tissue Using the Stromal-Vascular Fraction and Fibrin Hydrogels", TISSUE ENGINEERING: Part A, 2015, vol. 21, no. 7-8, pp. 1343-1353, in particular, abstract, fig. 1, 4 | 1-33 |
| Y | VERSEIJDEN, F. et al., "Vascularization of prevascularized and non-prevascularized fibrin-based human adipose tissue constructs after implantation in nude mice", JOURNAL OF TISSUE ENGINEERING AND REGENERATIVE MEDICINE, 2012, vol. 6, pp. 169-178, in particular, abstract, fig. 1 | 1-33 |
| A | JP 2003-116531 A (TOYOBO BOSEKI KABUSHIKI KAISHA) 22.04.2003 (2003-04-22) in particular, claims 1-8, examples, fig. 1 | 28-33 |
| A | WO 2005/093083 A1 (MAEDA, Kazuhisa) 06.10.2005 (2005-10-06) in particular, claims 1-29 | 28-33 |
| A | YAO, R. et al., "Biomimetic Injectable HUVEC-Adipocytes/Collagen/Alginate Microsphere Co-Cultures for Adipose Tissue Engineering", Biotechnology and Bioengineering, 2013, vol. 110, no. 5, pp. 1430-1443, entire text | 1-33 |
| A | 中 康博、外 2 名，足場材料のマイクロ化による新規なボトムアップ組織構築法の創製，第 40 回日本バイオマテリアル学会大会予稿集，2018, p. 421, #2P-050, entire text, non-official translation (NAKA, Yasuhiro et al., "Creation of a new bottom-up tissue construction approach by microfabrication of scaffold materials", Preprints of the 40th conference of Japanese Society for Biomaterials) | 1-33 |
| A | 中 康博、外 2 名，コラーゲンファイバーの架橋による物理的安定性の向上と三次元組織構築への応用，第 67 回高分子学会予稿集，2018, vol. 67, no. 1, #2H11, entire text, non-official translation (NAKA, Yasuhiro et al., "Improvement of physical stability by cross-linking collagen fiber and application to three-dimensional tissue construction", The 67th Polymer Preprints, Japan) | 1-33 |

Form PCT/ISA/210 (continuation of second sheet) (January 2015)

| | **INTERNATIONAL SEARCH REPORT** | International application No. |
|---|---|---|
| | | PCT/JP2020/012476 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2018-518970 A (NATIONAL UNIVERSITY OF SINGAPORE) 19.07.2018 (2018-07-19) entire text | 1-33 |
| A | JP 2012-237668 A (SHISEIDO CO., LTD.) 06.12.2012 (2012-12-06) entire text | 1-33 |
| A | JP 2010-00026 A (SHISEIDO CO., LTD.) 07.01.2010 (2010-01-07) entire text | 1-33 |
| P,X | LIU, H. et al., "Collagen Microfibers Induce Blood Capillary Orientation and Open Vascular Lumen", ADVANCED BIOSYSTEMS, 12 March 2020, no. 2000038, pp. 1-8, DOI:10.1002/adbi.202000038, entire text | 1-33 |

Form PCT/ISA/210 (continuation of second sheet) (January 2015)

**INTERNATIONAL SEARCH REPORT**
Information on patent family members

International application No.

PCT/JP2020/012476

| Patent Documents referred in the Report | Publication Date | Patent Family | Publication Date |
|---|---|---|---|
| WO 2018/143286 A1 | 09 Aug. 2018 | US 2019/0351098 A1 in particular, claims 1-15, examples, fig. 5 EP 3578638 A1 CN 110050059 A | |
| JP 2003-116531 A | 22 Apr. 2003 | (Family: none) | |
| WO 2005/093083 A1 | 06 Oct. 2005 | (Family: none) | |
| JP 2018-518970 A | 19 Jul. 2018 | US 2018/0187162 A1 entire text WO 2016/209166 A1 EP 3310903 A1 CN 107849530 A | |
| JP 2012-237668 A | 06 Dec. 2012 | WO 2012/153813 A1 entire text | |
| JP 2010-00026 A | 07 Jan. 2010 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015072164 A
- WO 2017146124 A

- WO 2018143286 A

**Non-patent literature cited in the description**

- **J. SILHA et al.** Angiogenic factors are elevated in overweight and obese individuals. *International Journal of Obesity,* 2005, vol. 29, 1308-1314